# EUROPEAN PATENT SPECIFICATION

(11) **EP 4 424 685 B1**
(45) Date of publication and mention of the grant of the patent: **17.06.2026**
(21) Application number: 22886138.1
(22) Date of filing: 28.10.2022
(51) Int. Cl.: C07D 401/12, C07D 401/14, C07D 403/12, C07D 471/04, C07D 487/04, A61K 31/437, A61K 31/4985, A61K 31/53, A61P 35/00

(54) **2(1H)PYRIDONE DERIVATIVES AS PORCUPINE (PORCN) INHIBITORS FOR THE TREATMENT OF CANCER**
2(1H)PYRIDON-DERIVATE ALS PORCUPIN (PORCN) INHIBITOREN ZUR BEHANDLUNG VON KREBS
DÉRIVÉS 2(1H)PYRIDONE EN TANT QU'INHIBITEURS DE PORCUPINE (PORCN) POUR LE TRAITEMENT DE CANCER

(30) Priority: 29.10.2021 CN 202111272187; 07.05.2022 CN 202210495439
(43) Date of publication of application: 04.09.2024
(73) Proprietor: Jiangsu Kanion Pharmaceutical Co., Ltd., Jiangsu 222047 (CN)
(72) Inventor: XU, Xiongbin, Shanghai 200131 (CN); CHEN, Yi, Shanghai 200131 (CN); DONG, Guang, Shanghai 200131 (CN); WU, Lingyun, Shanghai 200131 (CN); CHEN, Shuhui, Shanghai 200131 (CN)
(74) Representative: Mewburn Ellis LLP
(86) International application number: PCT/CN2022/128336
(87) International publication number: WO 2023/072263

(56) References cited:
- WO-A1-2017/123058
- WO-A1-2021/004467
- WO-A2-2011/082267
- CN-A- 102 369 187
- CN-A- 105 452 248

## Description

### TECHNICAL FIELD

The present disclosure relates to a class of 5-substituted pyridine-2(1H)-ketone compounds and a use thereof, and specifically relates to a compound of formula (X) or a pharmaceutically acceptable salt thereof.

### BACKGROUND

Porcupine (PORCN) protein is an acyltransferase that activates the extracellular secretion of Wnt protein through palmitoylation of WNT protein. The extracellular Wnt protein binds to the Frizzled receptor on the cell membrane, activating the Wnt/β-Catenin pathway, causing β-Catenin to accumulate and enter the cell nucleus. It binds to the TCF/LEF transcription factor in the nucleus, regulates the transcription of downstream target genes, and promotes the proliferation and activation of tumor cells.

Many tumors (including gastric cancer, colorectal cancer, liver cancer, and pancreatic cancer, etc.) have overactive WNT/*β*-Catenin signaling pathways. Porcupine inhibitors inhibit the activity of Porcupine protein, which can block the palmitoylation and extracellular secretion of Wnt protein, thereby inhibiting the abnormal activation of the Wnt/β-Catenin signaling pathway, and thus inhibiting the proliferation of various tumor cells. Currently, porcupine inhibitors entering the clinical trial stage include LGK974 (the patent application for this compound is WO2010101849) and ETC-1922159 (the patent application for this compound is WO2014189466). These porcupine inhibitors under clinical development have problems such as rapid metabolism, short half-life, and severe side effects. In view of this, developing new porcupine inhibitors to regulate Wnt/β-Catenin pathway signaling has important clinical value and social significance. 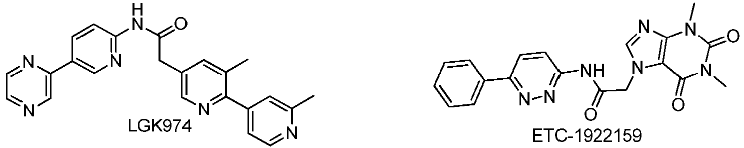 WO2021004467A1 discloses a compound of formula I acting as a porcupine inhibitor: 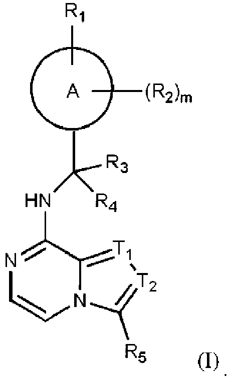 WO2011082267A2 discloses substituted triazolo-pyrazine compounds of formula I, II or III and their use in treating cell proliferative disorders:

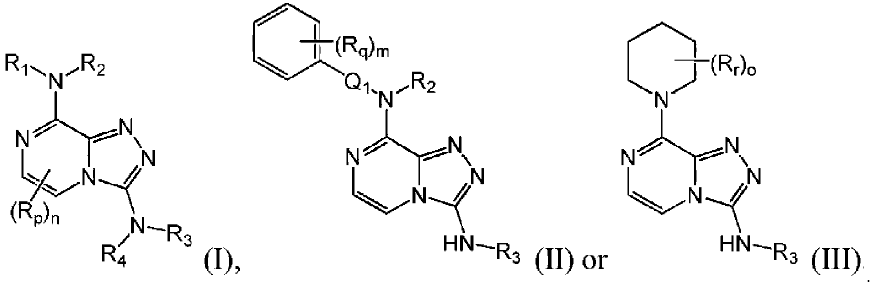

### CONTENT OF THE PRESENT INVENTION

In one aspect, the present disclosure provides a compound of formula (X) or a pharmaceutically acceptable salt thereof, 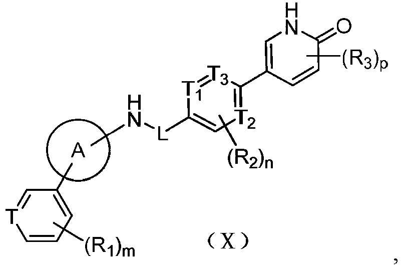 wherein
T is selected from CR or N;
T₁ is selected from CH or N;
T₂ is selected from CH or N;
T₃ is selected from CH or N;
L is selected from a single bond and -C(R₄R₅)-;
ring A is 9- to 10-membered heteroaryl, wherein the 9- to 10-membered heteroaryl is optionally substituted by 1, 2, or 3 Rₐ;
each R₁ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{b};
each R₂ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{c};
each R₃ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{d};
R₄ and R₅ are each independently selected from H and C₁₋₃ alkyl, wherein the C₁₋₃ alkyl is optionally substituted by 1, 2, or 3 Rₑ;
R is selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{f};
each Rₐ is independently selected from F, Cl, Br, I, -OH, -NH₂, and -CN;
each R_{b} is independently selected from F, Cl, Br, I, -OH, -NH₂, and -CN;
each R_{c} is independently selected from F, Cl, Br, I, -OH, -NH₂, and -CN;
each R_{d} is independently selected from F, Cl, Br, I, -OH, -NH₂, and -CN;
each Rₑ is independently selected from F, Cl, Br, I, -OH, -NH₂, and -CN;
each R_{f} is independently selected from F, Cl, Br, I, -OH, -NH₂, and -CN;
m is selected from 0, 1, 2, and 3;
n is selected from 0, 1, 2, and 3;
p is selected from 0, 1, 2, and 3;
"hetero" in the 9- to 10-membered heteroaryl represents 1, 2, 3, or 4 heteroatoms or heteroatom groups independently selected from -O-, -S-, and -N-.

In one aspect, the present disclosure provides a compound of formula (I) or a pharmaceutically acceptable salt thereof, 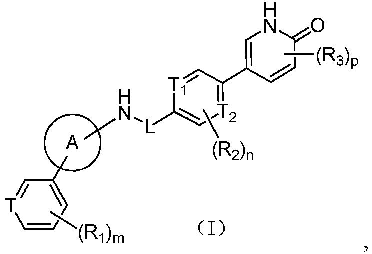 wherein
T is selected from CR or N;
T₁ is selected from CH or N;
T₂ is selected from CH or N;
L is selected from a single bond and -C(R₄R₅)-;
ring A is 9- to 10-membered heteroaryl, wherein the 9- to 10-membered heteroaryl is optionally substituted by 1, 2, or 3 Rₐ;
each R₁ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{b};
each R₂ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{c};
each R₃ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{d};
R₄ and R₅ are each independently selected from H and C₁₋₃ alkyl, wherein the C₁₋₃ alkyl is optionally substituted by 1, 2, or 3 Rₑ;
R is selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{f};
each Rₐ is independently selected from F, Cl, Br, I, -OH, -NH₂, and -CN;
each R_{b} is independently selected from F, Cl, Br, I, -OH, -NH₂, and -CN;
each R_{c} is independently selected from F, Cl, Br, I, -OH, -NH₂, and -CN;
each R_{d} is independently selected from F, Cl, Br, I, -OH, -NH₂, and -CN;
each Rₑ is independently selected from F, Cl, Br, I, -OH, -NH₂, and -CN;
each R_{f} is independently selected from F, Cl, Br, I, -OH, -NH₂, and -CN;
m is selected from 0, 1, 2, and 3;
n is selected from 0, 1, 2, and 3;
p is selected from 0, 1, 2, and 3;
"hetero" in the 9- to 10-membered heteroaryl represents 1, 2, 3, or 4 heteroatoms or heteroatom groups independently selected from -O-, -S-, and -N-.

In some embodiments of the present disclosure, the compound has a structure of formula (X-1): 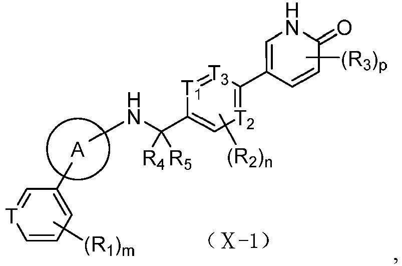 wherein ring A, R₁, R₂, R₃, R₄, R₅, T, T₁, T₂, T₃, m, n, and p are as defined in the present disclosure.

In some embodiments of the present disclosure, the compound has a structure of formula (I-1): 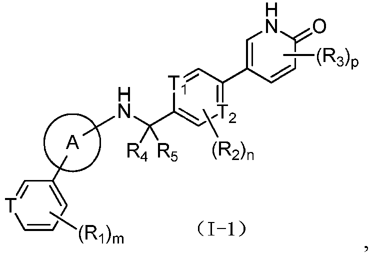 wherein ring A, R₁, R₂, R₃, R₄, R₅, T, T₁, T₂, m, n, and p are as defined in the present disclosure.

In some embodiments of the present disclosure, each R₁ is independently selected from H, F, Cl, Br, I, -CN, -OH, -NH₂, -CH₃, and -OCH₃, wherein the -CH₃ and -OCH₃ are each independently and optionally substituted by 1, 2, or 3 R_{b}, and R_{b} and other variables are as defined in the present disclosure.

In some embodiments of the present disclosure, the T is selected from CR, and R and other variables are as defined in the present disclosure.

In some embodiments of the present disclosure, the T is selected from N, and other variables are as defined in the present disclosure.

In some embodiments of the present disclosure, the T₁ is selected from CH, and other variables are as defined in the present disclosure.

In some embodiments of the present disclosure, the T₁ is selected from N, and other variables are as defined in the present disclosure.

In some embodiments of the present disclosure, the T₂ is selected from CH, and other variables are as defined in the present disclosure.

In some embodiments of the present disclosure, the T₂ is selected from N, and other variables are as defined in the present disclosure.

In some embodiments of the present disclosure, the T₃ is selected from CH, and other variables are as defined in the present disclosure.

In some embodiments of the present disclosure, the T₃ is selected from N, and other variables are as defined in the present disclosure.

In some embodiments of the present disclosure, each R₁ is independently selected from H, F, Cl, Br, I, -CN, -OH, -NH₂, and -CH₃, wherein the -CH₃ is optionally substituted by 1, 2, or 3 R_{b}, and R_{b} and other variables are as defined in the present disclosure.

In some embodiments of the present disclosure, each R₁ is independently selected from H, F, -CH₃, and -OCH₃, and other variables are as defined in the present disclosure.

In some embodiments of the present disclosure, each R₁ is independently selected from H and F, and other variables are as defined in the present disclosure.

In some embodiments of the present disclosure, each R₁ is independently selected from F, and other variables are as defined in the present disclosure. In some embodiments of the present disclosure, each R₂ is independently selected from H, and other variables are as defined in the present disclosure.

In some embodiments of the present disclosure, each R₃ is independently selected from H, F, Cl, Br, I, -CN, -OH, -NH₂, and -CH₃, wherein the -CH₃ is optionally substituted by 1, 2, or 3 R_{d}, and R_{b} and other variables are as defined in the present disclosure.

In some embodiments of the present disclosure, each R₃ is independently selected from -CH₃, and other variables are as defined in the present disclosure.

In some embodiments of the present disclosure, the R₄ and R₅ are each independently selected from H and -CH₃, wherein the -CH₃ is optionally substituted by 1, 2, or 3 Rₑ, and Rₑ and other variables are as defined in the present disclosure.

In some embodiments of the present disclosure, the R₄ and R₅ are each independently selected from H, and other variables are as defined in the present disclosure.

In some embodiments of the present disclosure, the R is selected from H, F, Cl, Br, I, -CN, -OH, -NH₂, and -CH₃, wherein the -CH₃ is optionally substituted by 1, 2, or 3 R_{f}, and R_{f} and other variables are as defined in the present disclosure.

In some embodiments of the present disclosure, the R is selected from H and F, and other variables are as defined in the present disclosure.

In some embodiments of the present disclosure, the R is selected from F, and other variables are as defined in the present disclosure.

In some embodiments of the present disclosure, the m is selected from 0, and other variables are as defined in the present disclosure.

In some embodiments of the present disclosure, the m is selected from 1, and other variables are as defined in the present disclosure.

In some embodiments of the present disclosure, the m is selected from 2, and other variables are as defined in the present disclosure.

In some embodiments of the present disclosure, the ring A is selected from 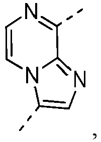 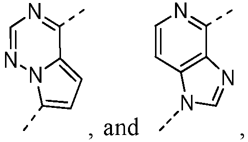 wherein 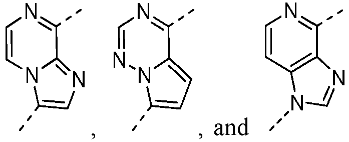 are each independently and optionally substituted by 1, 2, or 3 Rₐ, and Rₐ and other variables are as defined in the present disclosure.

In some embodiments of the present disclosure, the ring A is selected from 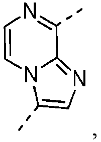 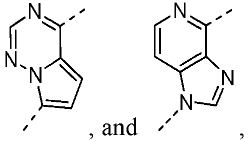 and other variables are as defined in the present disclosure.

In some embodiments of the present disclosure, the compound has a structure of formula (X-2) or (X-3): 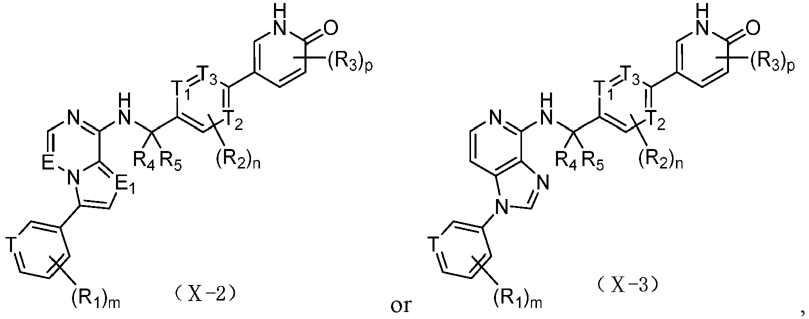 wherein
E is selected from CH and N;
E₁ is selected from CH and N;
R₁, R₂, R₃, R₄, R₅, T, T₁, T₂, T₃, m, n, and p are as defined in the present disclosure.

In some embodiments of the present disclosure, the compound has a structure of formula (I-2) or (I-3): 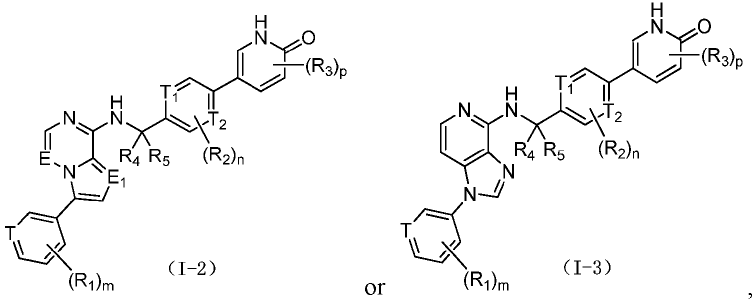 wherein
E is selected from CH and N;
E₁ is selected from CH and N;
R₁, R₂, R₃, R₄, R₅, T, T₁, T₂, m, n, and p are as defined in the present disclosure.

In some embodiments of the present disclosure, the compound has a structure of formula (X-4), (X-5), or (X-6): 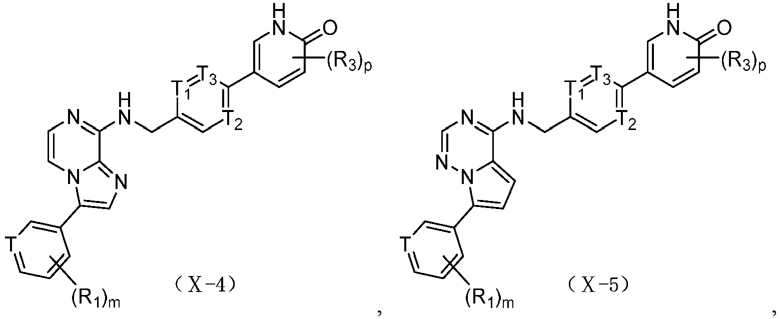 or 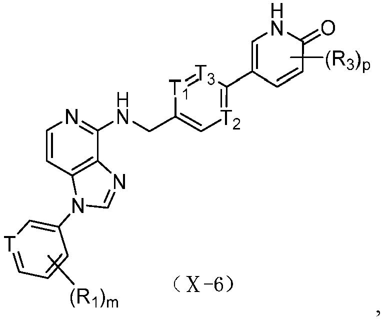 wherein R₁, R₃, T, T₁, T₂, T₃, m, and p are as defined in the present disclosure.

In some embodiments of the present disclosure, the compound has a structure of formula (I-4), (I-5), or (I-6): 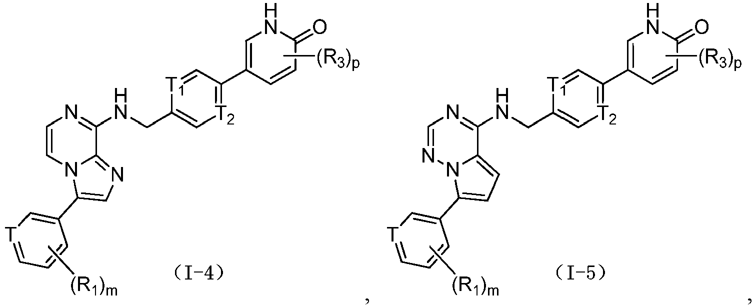 or 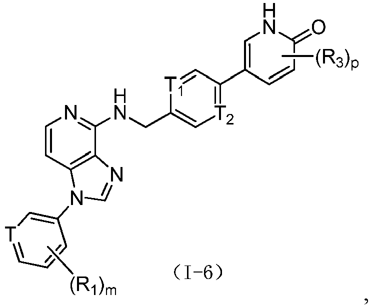 wherein R₁, R₃, T, T₁, T₂, m, and p are as defined in the present disclosure.

In some embodiments of the present disclosure, the compound has a structure of formulas (X-7), (X-8), and (X-9): 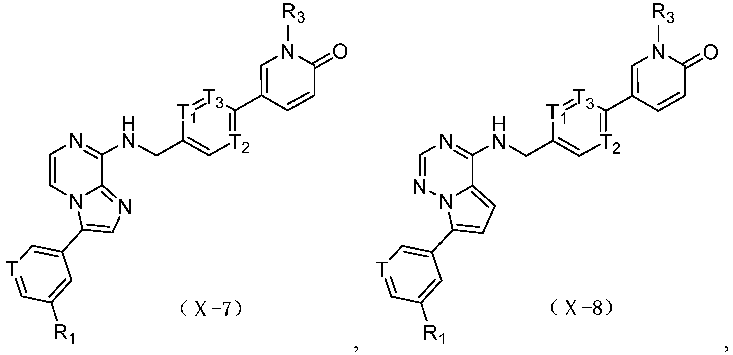 or 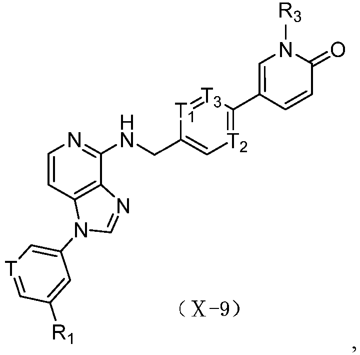 wherein R₁, R₃, T, T₁, T₂, and T₃ are as defined in the present disclosure.

In some embodiments of the present disclosure, the compound has a structure of formulas (I-7), (I-8), and (I-9): 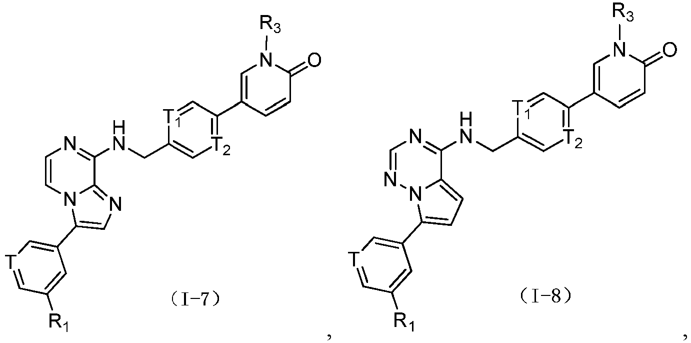 or 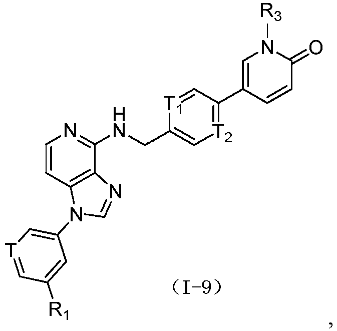 wherein R₁, R₃, T, T₁, and T₂ are as defined in the present disclosure.

In some embodiments of the present disclosure, the compound has a structure of formula (X-4A), (X-4B), (X-4C), (X-4D), (X-5A), or (X-6A): 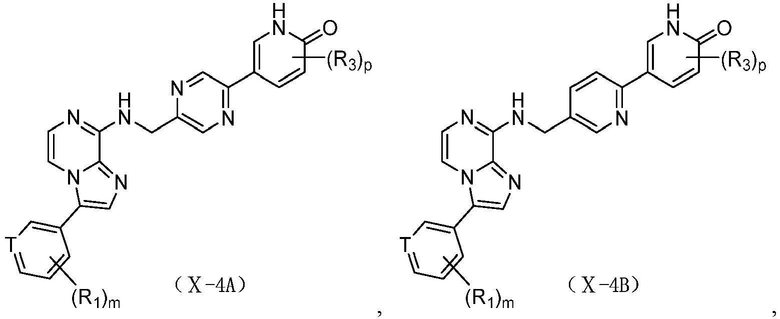 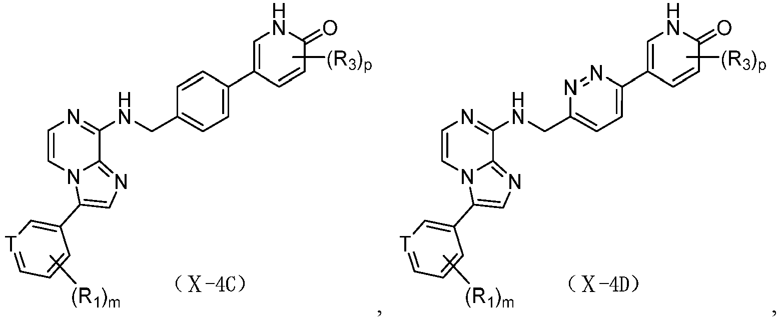 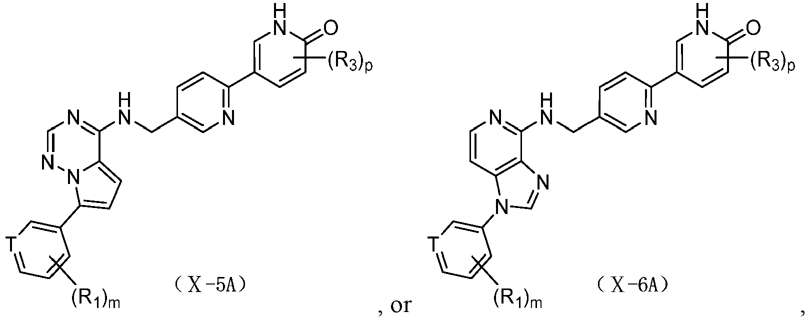 wherein R₁, R₃, T, m, and p are as defined in the present disclosure.

In some embodiments of the present disclosure, the compound has a structure of formula (X-4A-1), (X-4B-1), (X-4C-1), (X-4D-1), (X-5A-1), or (X-6A-1): 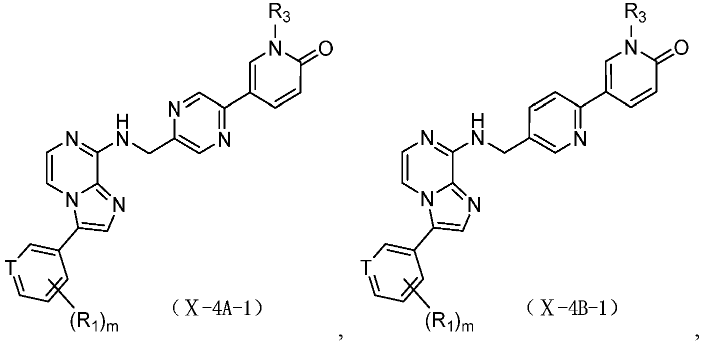 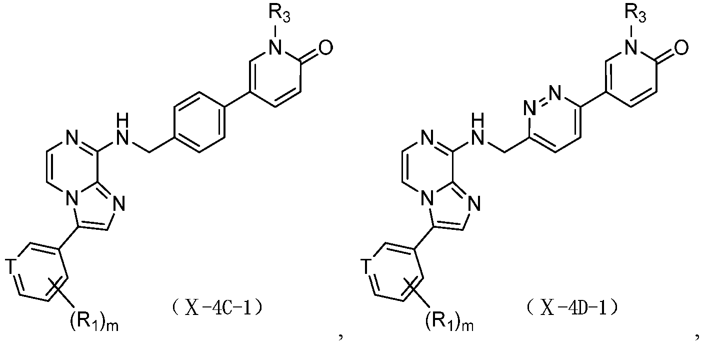 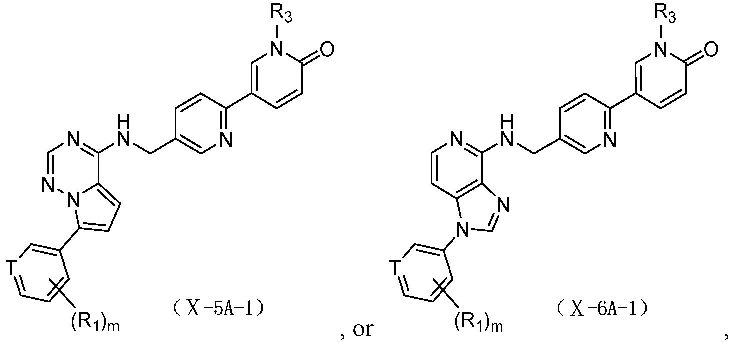 wherein R₁, R₃, T, and m are as defined in the present disclosure.

There are still some embodiments of the present disclosure which are obtained by any combination of the above variables.

The present disclosure also provides a compound of the following formula or a pharmaceutically acceptable salt thereof,

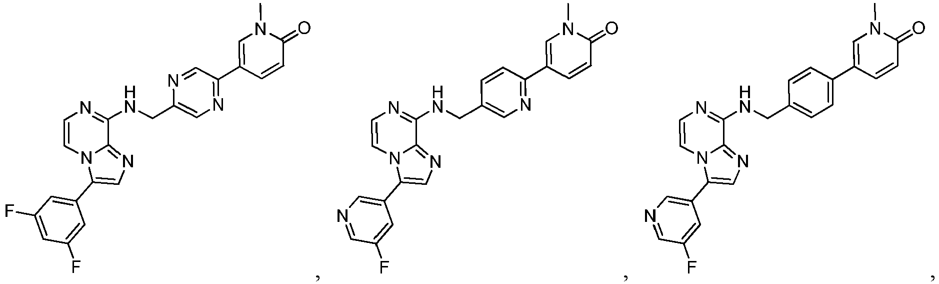

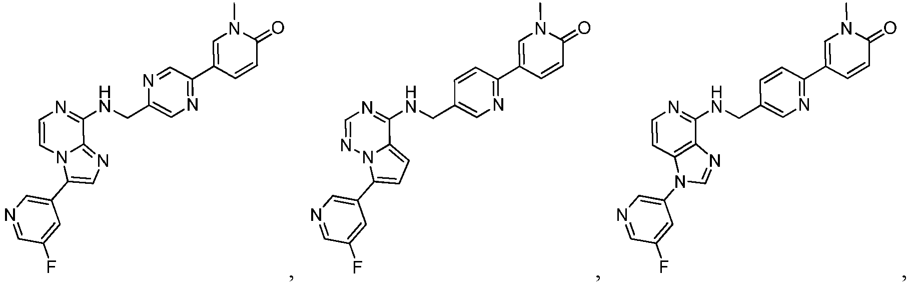

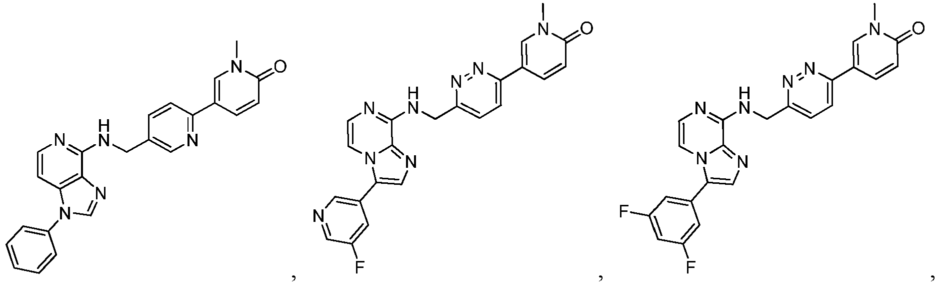

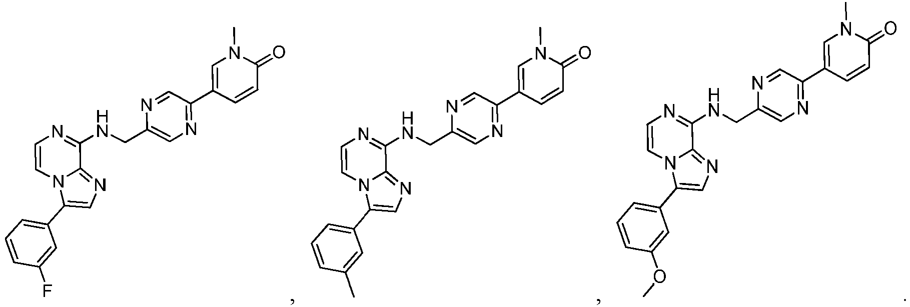

The present disclosure also provides a use of the compound or the pharmaceutically acceptable salt thereof in the manufacture of a porcupine inhibitor medicament.

The present disclosure also provides a use of the compound or the pharmaceutically acceptable salt thereof in the manufacture of a medicament for treating pancreatic cancer, colorectal cancer, and gastric cancer.

The present disclosure provides a crystal form A of compound 2, wherein the crystal form A has an X-ray powder diffraction pattern comprising characteristic diffraction peaks at the following 2θ angles: 7.45 ± 0.20°, 13.53 ± 0.20°, 13.94 ± 0.20°, and 15.93 ± 0.20°;

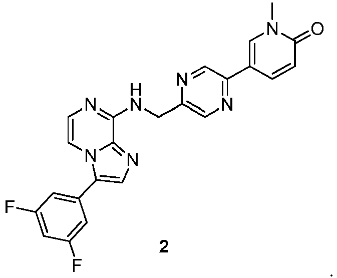

In some embodiments of the present disclosure, the crystal form A is characterized in that the X-ray powder diffraction pattern of the crystal form A comprises characteristic **diffraction** peaks at the following 2θ angles: 7.45 ± 0.20°, 10.56 ± 0.20°, 13.53 ± 0.20°, 13.94 ± 0.20°, 14.86 ± 0.20°, 15.93 ± 0.20°, and 17.96 ± 0.20°.

In some embodiments of the present disclosure, the crystal form A is characterized in that the X-ray powder diffraction pattern of the crystal form A, expressed in 2θ angle, comprises at least 4, 5, 6, or 7 characteristic diffraction peaks selected from the following: 7.45 ± 0.20°, 10.56 ± 0.20°, 13.53 ± 0.20°, 13.94 ± 0.20°, 14.86 ± 0.20°, 15.93 ± 0.20°, and 17.96 ± 0.20°.

In some embodiments of the present disclosure, the X-ray powder diffraction pattern of the crystal form A comprises characteristic diffraction peaks at the following 2θ angles: 4.96 ± 0.20°, 7.45 ± 0.20°, 10.56 ± 0.20°, 13.53 ± 0.20°, 13.94 ± 0.20°, 14.86 ± 0.20°, 15.93 ± 0.20°, 17.96 ± 0.20°, 19.87 ± 0.20°, 20.90 ± 0.20°, 25.38 ± 0.20°, and 28.02 ± 0.20°.

In some embodiments of the present disclosure, the crystal form A is characterized in that the X-ray powder diffraction pattern of the crystal form A, expressed in 2θ angle, comprises at least 4, 5, 6, 7, 8, 9, 10, 11, or 12 characteristic diffraction peaks selected from the following: 4.96 ± 0.20°, 7.45 ± 0.20°, 10.56 ± 0.20°, 13.53 ± 0.20°, 13.94 ± 0.20°, 14.86 ± 0.20°, 15.93 ± 0.20°, 17.96 ± 0.20°, 19.87 ± 0.20°, 20.90 ± 0.20°, 25.38 ± 0.20°, and 28.02 ± 0.20°.

In some embodiments of the present disclosure, the X-ray powder diffraction pattern of the crystal form A comprises characteristic diffraction peaks at the following 2θ angles: 4.96 ± 0.20°, 7.45 ± 0.20°, 10.56 ± 0.20°, 13.53 ± 0.20°, 13.94 ± 0.20°, 14.86 ± 0.20°, 15.93 ± 0.20°, 17.96 ± 0.20°, 19.06 ± 0.20°, 19.87 ± 0.20°, 20.90 ± 0.20°, 24.11 ± 0.20°, 24.97 ± 0.20°, 25.38 ± 0.20°, and 28.02 ± 0.20°.

In some embodiments of the present disclosure, the X-ray powder diffraction pattern of the crystal form A comprises characteristic diffraction peaks at the following 2θ angles: 4.96 ± 0.20°, 7.45 ± 0.20°, 10.56 ± 0.20°, 13.53 ± 0.20°, 13.94 ± 0.20°, 14.86 ± 0.20°, 15.93 ± 0.20°, 17.56 ± 0.20°, 17.96 ± 0.20°, 19.06 ± 0.20°, 19.87 ± 0.20°, 20.90 ± 0.20°, 21.21 ± 0.20°, 21.90 ± 0.20°, 22.63 ± 0.20°, 24.11 ± 0.20°, 24.97 ± 0.20°, 25.38 ± 0.20°, 25.96 ± 0.20°, 27.14 ± 0.20°, 28.02 ± 0.20°, 28.73 ± 0.20°, 29.94 ± 0.20°, 30.89 ± 0.20°, 32.50 ± 0.20°, 34.01 ± 0.20°, 35.05 ± 0.20°, 35.95 ± 0.20°, 37.54 ± 0.20°, 39.08 ± 0.20°.

In some embodiments of the present disclosure, the X-ray powder diffraction pattern of the crystal form A comprises characteristic diffraction peaks at the following 2θ angles: 4.96°, 7.45°, 10.56°, 13.53°, 13.94°, 14.86°, 15.93°, 17.56°, 17.96°, 19.06°, 19.87°, 20.90°, 21.21°, 21.90°, 22.63°, 24.11°, 24.97°, 25.38°, 25.96°, 27.14°, 28.02°, 28.73°, 29.94°, 30.89°, 32.50°, 34.01°, 35.05°, 35.95°, 37.54°, and 39.08°.

In some embodiments of the present disclosure, the X-ray powder diffraction pattern of the crystal form A comprises characteristic diffraction peaks at the following 2θ angles: 13.53 ± 0.20°, 13.94 ± 0.20°, 15.93 ± 0.20°, and/or 4.96 ± 0.20°, and/or 7.45 ± 0.20°, and/or 10.56 ± 0.20°, and/or 14.86 ± 0.20°, and/or 17.56 ± 0.20°, and/or 17.96 ± 0.20°, and/or 19.06 ± 0.20°, and/or 19.87 ± 0.20°, and/or 20.90 ± 0.20°, and/or 21.21 ± 0.20°, and/or 21.90 ± 0.20°, and/or 22.63 ± 0.20°, and/or 24.11 ± 0.20°, and/or 24.97 ± 0.20°, and/or 25.38 ± 0.20°, and/or 25.96 ± 0.20°, and/or 27.14 ± 0.20°, and/or 28.02 ± 0.20°, and/or 28.73 ± 0.20°, and/or 29.94 ± 0.20°, and/or 30.89 ± 0.20°, and/or 32.50 ± 0.20°, and/or 34.01 ± 0.20°, and/or 35.05 ± 0.20°, and/or 35.95 ± 0.20°, and/or 37.54 ± 0.20°, and/or 39.08 ± 0.20°.

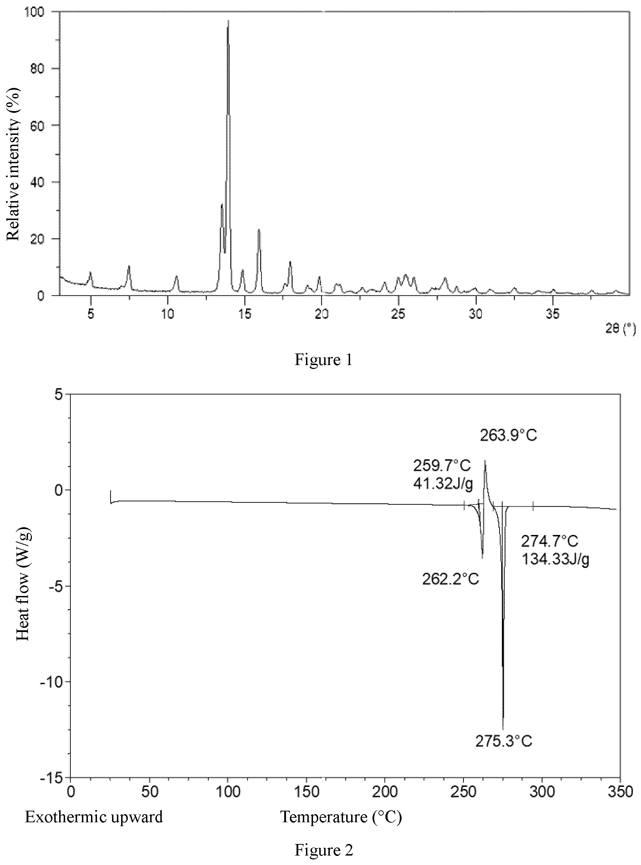

In some embodiments of the present disclosure, the XRPD pattern of the crystal form A is basically as shown in Figure 1.

In some embodiments of the present disclosure, XRPD pattern analysis data of the crystal form A are shown in Table 1.

**Table 1 XRPD pattern analysis data of crystal form A of compound 2**

| No. | 2θ angle [°] | Peak height [cts] | Left full width at half maximum [°2θ] | Interplanar spacing [Å] | Relative intensity [%] |
|---|---|---|---|---|---|
| 1 | 4.96 | 798.52 | 0.1279 | 17.80 | 5.32 |
| 2 | 7.45 | 1349.94 | 0.1535 | 11.87 | 9.00 |
| 3 | 10.56 | 873.60 | 0.1791 | 8.37 | 5.83 |
| 4 | 13.53 | 4986.93 | 0.1791 | 6.55 | 33.25 |
| 5 | 13.94 | 14996.96 | 0.1791 | 6.35 | 100.00 |
| 6 | 14.86 | 1270.50 | 0.1791 | 5.96 | 8.47 |
| 7 | 15.93 | 3547.78 | 0.1791 | 5.56 | 23.66 |
| 8 | 17.56 | 432.50 | 0.1791 | 5.05 | 2.88 |
| 9 | 17.96 | 1770.00 | 0.1791 | 4.94 | 11.80 |
| 10 | 19.06 | 435.51 | 0.1535 | 4.66 | 2.90 |
| 11 | 19.87 | 899.45 | 0.2047 | 4.47 | 6.00 |
| 12 | 20.90 | 460.45 | 0.1535 | 4.25 | 3.07 |
| 13 | 21.21 | 423.35 | 0.1535 | 4.19 | 2.82 |
| 14 | 21.90 | 104.94 | 0.2558 | 4.06 | 0.70 |
| 15 | 22.63 | 291.20 | 0.1279 | 3.93 | 1.94 |
| 16 | 24.11 | 596.83 | 0.1791 | 3.69 | 3.98 |
| 17 | 24.97 | 871.02 | 0.2047 | 3.57 | 5.81 |
| 18 | 25.38 | 979.35 | 0.2047 | 3.51 | 6.53 |
| 19 | 25.96 | 879.75 | 0.1791 | 3.43 | 5.87 |
| 20 | 27.14 | 280.14 | 0.2047 | 3.29 | 1.87 |
| 21 | 28.02 | 877.39 | 0.2047 | 3.18 | 5.85 |
| 22 | 28.73 | 379.96 | 0.1535 | 3.11 | 2.53 |
| 23 | 29.94 | 275.89 | 0.4093 | 2.98 | 1.84 |
| 24 | 30.89 | 224.53 | 0.2047 | 2.89 | 1.50 |
| 25 | 32.50 | 309.77 | 0.2047 | 2.75 | 2.07 |
| 26 | 34.01 | 178.62 | 0.2558 | 2.64 | 1.19 |
| 27 | 35.05 | 248.70 | 0.1535 | 2.56 | 1.66 |
| 28 | 35.95 | 67.09 | 0.3070 | 2.50 | 0.45 |
| 29 | 37.54 | 224.08 | 0.2303 | 2.40 | 1.49 |
| 30 | 39.08 | 170.32 | 0.2047 | 2.30 | 1.14 |

In some embodiments of the present disclosure, the crystal form A has a differential scanning calorimetry curve comprising onsets of endothermic peaks at 259.7°C ± 5°C and 274.7°C ± 5°C.

In some embodiments of the present disclosure, the DSC pattern of the crystal form A is basically as shown in Figure 2.

In some embodiments of the present disclosure, the crystal form A has a thermogravimetric analysis curve with a weight loss of 0.80% at 240°C ± 3°C.

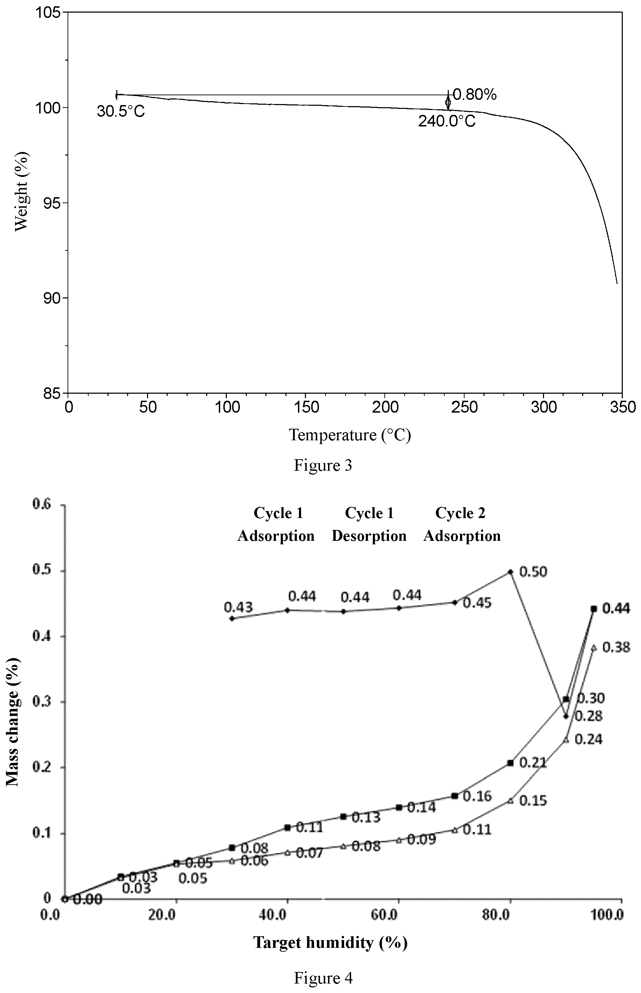

In some embodiments of the present disclosure, the TGA pattern of the crystal form A is basically as shown in Figure 3.

In some embodiments of the present disclosure, the DVS isotherm pattern of the crystal form A is basically as shown in Figure 4.

The present disclosure also provides a crystal form B of compound 2, which is not part of the present invention, wherein the crystal form B has an X-ray powder diffraction pattern comprising characteristic diffraction peaks at the following 2θ angles: 13.43 ± 0.20°, 14.07 ± 0.20°, and 15.07 ± 0.20°;

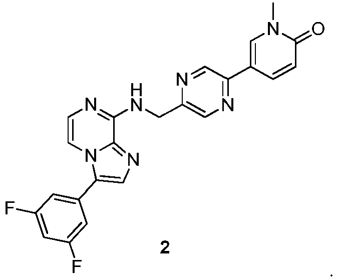

In some embodiments of the present disclosure, the X-ray powder diffraction pattern of the crystal form B comprises characteristic diffraction peaks at the following 2θ angles: 11.72 ± 0.20°, 13.43 ± 0.20°, 14.07 ± 0.20°, 15.07 ± 0.20°, 16.39 ± 0.20°, 18.21 ± 0.20°, 22.53 ± 0.20°, and 25.23 ± 0.20°.

In some embodiments of the present disclosure, the crystal form B is characterized in that the X-ray powder diffraction pattern of the crystal form B, expressed in 2θ angle, comprises at least 4, 5, 6, 7, or 8 characteristic diffraction peaks selected from the following: 11.72 ± 0.20°, 13.43 ± 0.20°, 14.07 ± 0.20°, 15.07 ± 0.20°, 16.39 ± 0.20°, 18.21 ± 0.20°, 22.53 ± 0.20°, and 25.23 ± 0.20°.

In some embodiments of the present disclosure, the X-ray powder diffraction pattern of the crystal form B comprises characteristic diffraction peaks at the following 2θ angles: 4.86 ± 0.20°, 7.05 ± 0.20°, 11.72 ± 0.20°, 13.43 ± 0.20°, 14.07 ± 0.20°, 14.54 ± 0.20°, 15.07 ± 0.20°, 16.39 ± 0.20°, 18.21 ± 0.20°, 19.67 ± 0.20°, 22.53 ± 0.20°, and 25.23 ± 0.20°.

In some embodiments of the present disclosure, the crystal form B is characterized in that the X-ray powder diffraction pattern of the crystal form B, expressed in 2θ angle, comprises at least 4, 5, 6, 7, 8, 9, 10, 11, or 12 characteristic diffraction peaks selected from the following: **4.86 ± 0.20°,** 7.05 ± 0.20°, 11.72 ± 0.20°, 13.43 ± 0.20°, 14.07 ± 0.20°, 14.54 ± 0.20°, 15.07 ± 0.20°, 16.39 ± 0.20°, 18.21 ± 0.20°, 19.67 ± 0.20°, 22.53 ± 0.20°, and 25.23 ± 0.20°.

In some embodiments of the present disclosure, the X-ray powder diffraction pattern of the crystal form B comprises characteristic diffraction peaks at the following 2θ angles: 4.86 ± 0.20°, 7.05 ± 0.20°, 11.72 ± 0.20°, 13.43 ± 0.20°, 14.07 ± 0.20°, 14.50 ± 0.20°, 15.07 ± 0.20°, 16.39 ± 0.20°, 18.21 ± 0.20°, 19.67 ± 0.20°, 22.53 ± 0.20°, 23.24 ± 0.20°, 24.28 ± 0.20°, 25.23 ± 0.20°, 28.32 ± 0.20°, and 29.39 ± 0.20°.

In some embodiments of the present disclosure, the X-ray powder diffraction pattern of the crystal form B comprises characteristic diffraction peaks at the following 2θ angles: 4.86 ± 0.20°, 7.05 ± 0.20°, 11.72 ± 0.20°, 13.43 ± 0.20°, 14.07 ± 0.20°, 14.50 ± 0.20°, 15.07 ± 0.20°, 15.97 ± 0.20°, 16.39 ± 0.20°, 18.21 ± 0.20°, 19.39 ± 0.20°, 19.67 ± 0.20°, 21.18 ± 0.20°, 22.53 ± 0.20°, 23.24 ± 0.20°, 24.28 ± 0.20°, 25.23 ± 0.20°, 25.79 ± 0.20°, 26.46 ± 0.20°, 27.20 ± 0.20°, 27.90 ± 0.20°, 28.32 ± 0.20°, 28.90 ± 0.20°, 29.39 ± 0.20°, 31.70 ± 0.20°, 32.54 ± 0.20°, 33.50 ± 0.20°, 34.34 ± 0.20°, 34.90 ± 0.20°, 37.53 ± 0.20°, and 38.96 ± 0.20°.

In some embodiments of the present disclosure, the X-ray powder diffraction pattern of the crystal form B comprises characteristic diffraction peaks at the following 2θ angles: 4.86 ± 0.10°, 7.05 ± 0.10°, 11.72 ± 0.10°, 13.43 ± 0.10°, 14.07 ± 0.10°, 14.50 ± 0.10°, 15.07 ± 0.10°, 15.97 ± 0.10°, 16.39 ± 0.10°, 18.21 ± 0.10°, 19.39 ± 0.10°, 19.67 ± 0.10°, 21.18 ± 0.10°, 22.53 ± 0.10°, 23.24 ± 0.10°, 24.28 ± 0.10°, 25.23 ± 0.10°, 25.79 ± 0.10°, 26.46 ± 0.10°, 27.20 ± 0.10°, 27.90 ± 0.10°, 28.32 ± 0.10°, 28.90 ± 0.10°, 29.39 ± 0.10°, 31.70 ± 0.10°, 32.54 ± 0.10°, 33.50 ± 0.10°, 34.34 ± 0.10°, 34.90 ± 0.10°, 37.53 ± 0.10°, and 38.96 ± 0.10°.

In some embodiments of the present disclosure, the X-ray powder diffraction pattern of the crystal form B comprises characteristic diffraction peaks at the following 2θ angles: 4.86°, 7.05°, 11.72°, 13.43°, 14.07°, 14.50°, 15.07°, 15.97°, 16.39°, 18.21°, 19.39°, 19.67°, 21.18°, 22.53°, 23.24°, 24.28°, 25.23°, 25.79°, 26.46°, 27.20°, 27.90°, 28.32°, 28.90°, 29.39°, 31.70°, 32.54°, 33.50°, 34.34°, 34.90°, 37.53°, and 38.96°.

In some embodiments of the present disclosure, the X-ray powder diffraction pattern of the crystal form B comprises characteristic diffraction peaks at the following 2θ angles: 13.43 ± 0.20°, 14.07 ± 0.20°, 15.07 ± 0.20°, and/or 4.86 ± 0.20°, and/or 7.05 ± 0.20°, and/or 11.72 ± 0.20°, and/or 14.50 ± 0.20°, and/or 15.97 ± 0.20°, and/or 16.39 ± 0.20°, and/or 18.21 ± 0.20°, and/or 19.39 ± 0.20°, and/or 19.67 ± 0.20°, and/or 21.18 ± 0.20°, and/or 22.53 ± 0.20°, and/or 23.24 ± 0.20°, and/or 24.28 ± 0.20°, and/or 25.23 ± 0.20°, and/or 25.79 ± 0.20°, and/or 26.46 ± 0.20°, and/or 27.20 ± 0.20°, and/or 27.90 ± 0.20°, and/or 28.32 ± 0.20°, and/or 28.90 ± 0.20°, and/or 29.39 ± 0.20°, and/or 31.70 ± 0.20°, and/or 32.54 ± 0.20°, and/or 33.50 ± 0.20°, and/or 34.34 ± 0.20°, and/or 34.90 ± 0.20°, and/or 37.53 ± 0.20°, and/or 38.96 ± 0.20°.

In some embodiments of the present disclosure, the X-ray powder diffraction pattern of the crystal form B comprises characteristic diffraction peaks at the following 2θ angles: 13.43 ± 0.10°, 14.07 ± 0.10°, 15.07 ± 0.10°, and/or 4.86 ± 0.10°, and/or 7.05 ± 0.10°, and/or 11.72 ± 0.10°, and/or 14.50 ± 0.10°, and/or 15.97 ± 0.10°, and/or 16.39 ± 0.10°, and/or 18.21 ± 0.10°, and/or 19.39 ± 0.10°, and/or 19.67 ± 0.10°, and/or 21.18 ± 0.10°, and/or 22.53 ± 0.10°, and/or 23.24 ± 0.10°, and/or 24.28 ± 0.10°, and/or 25.23 ± 0.10°, and/or 25.79 ± 0.10°, and/or 26.46 ± 0.10°, and/or 27.20 ± 0.10°, and/or 27.90 ± 0.10°, and/or 28.32 ± 0.10°, and/or 28.90 ± 0.10°, and/or 29.39 ± 0.10°, and/or 31.70 ± 0.10°, and/or 32.54 ± 0.10°, and/or 33.50 ± 0.10°, and/or 34.34 ± 0.10°, and/or 34.90 ± 0.10°, and/or 37.53 ± 0.10°, and/or 38.96 ± 0.10°.

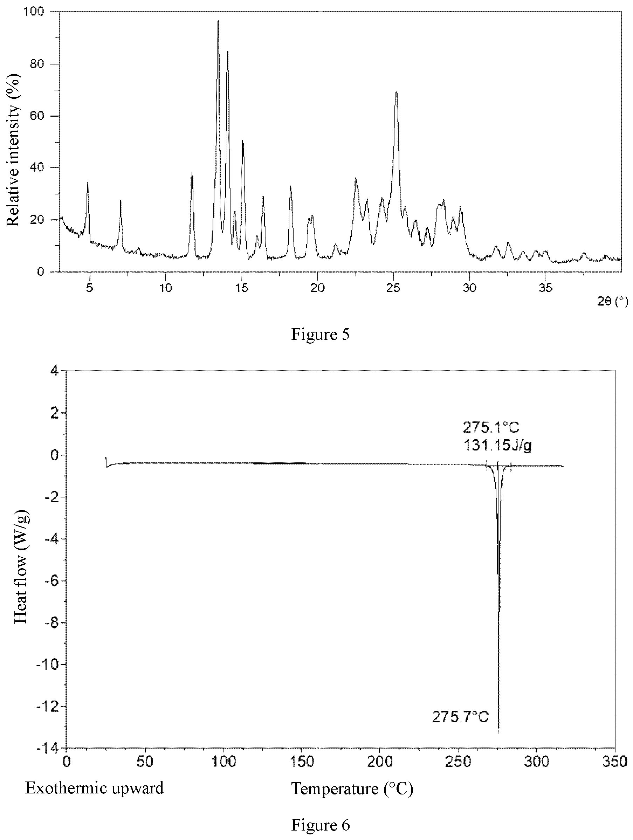

In some embodiments of the present disclosure, the XRPD pattern of the crystal form B is basically as shown in Figure 5.

In some embodiments of the present disclosure, XRPD pattern analysis data of the crystal form B are shown in Table 2.

**Table 2 XRPD pattern analysis data of crystal form B of compound 2**

| No. | 2θ angle [°] | Peak height [cts] | Left full width at half maximum [°2θ] | Interplanar spacing [Å] | Relative intensity [%] |
|---|---|---|---|---|---|
| 1 | 4.86 | 890.06 | 0.1279 | 18.16 | 24.09 |
| 2 | 7.05 | 755.03 | 0.1279 | 12.54 | 20.44 |
| 3 | 11.72 | 1301.15 | 0.1791 | 7.55 | 35.22 |
| 4 | 13.43 | 3694.53 | 0.1791 | 6.59 | 100.00 |
| 5 | 14.07 | 3186.73 | 0.2047 | 6.30 | 86.26 |
| 6 | 14.54 | 707.32 | 0.1279 | 6.09 | 19.15 |
| 7 | 15.07 | 1794.49 | 0.2047 | 5.88 | 48.57 |
| 8 | 15.97 | 331.05 | 0.1279 | 5.55 | 8.96 |
| 9 | 16.39 | 962.71 | 0.1791 | 5.41 | 26.06 |
| 10 | 18.21 | 1077.94 | 0.2303 | 4.87 | 29.18 |
| 11 | 19.39 | 576.90 | 0.1535 | 4.58 | 15.62 |
| 12 | 19.67 | 656.61 | 0.2047 | 4.51 | 17.77 |
| 13 | 21.18 | 195.42 | 0.2558 | 4.19 | 5.29 |
| 14 | 22.53 | 1165.11 | 0.1279 | 3.95 | 31.54 |
| 15 | 23.24 | 786.72 | 0.2047 | 3.83 | 21.29 |
| 16 | 24.28 | 824.02 | 0.2047 | 3.67 | 22.3 |
| 17 | 25.23 | 2427.67 | 0.2814 | 3.53 | 65.71 |
| 18 | 25.79 | 663.55 | 0.2047 | 3.45 | 17.96 |
| 19 | 26.46 | 495.08 | 0.307 | 3.37 | 13.40 |
| 20 | 27.20 | 393.61 | 0.2558 | 3.28 | 10.65 |
| 21 | 27.90 | 717.66 | 0.307 | 3.2 | 19.42 |
| 22 | 28.32 | 825.56 | 0.15 | 3.15 | 22.35 |
| 23 | 28.90 | 588.80 | 0.20 | 3.09 | 15.94 |
| 24 | 29.39 | 763.03 | 0.41 | 3.04 | 20.65 |
| 25 | 31.70 | 219.16 | 0.31 | 2.82 | 5.93 |
| 26 | 32.54 | 283.96 | 0.36 | 2.75 | 7.69 |
| 27 | 33.50 | 147.99 | 0.31 | 2.68 | 4.01 |
| 28 | 34.34 | 162.81 | 0.26 | 2.61 | 4.41 |
| 29 | 34.90 | 163.90 | 0.36 | 2.57 | 4.44 |
| 30 | 37.52 | 122.41 | 0.20 | 2.40 | 3.31 |
| 31 | 38.96 | 50.80 | 0.31 | 2.31 | 1.37 |

In some embodiments of the present disclosure, the crystal form B has a differential scanning calorimetry curve comprising an onset of an endothermic peak at 275.1°C ± 5°C.

In some embodiments of the present disclosure, the DSC pattern of the crystal form B is basically as shown in Figure 6.

In some embodiments of the present disclosure, the crystal form B has a thermogravimetric analysis curve with a weight loss of 0.77% at 260°C ± 3°C.

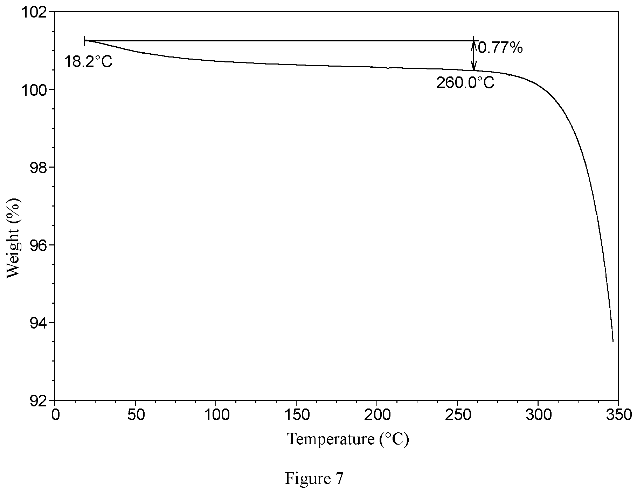

In some embodiments of the present disclosure, the TGA pattern of the crystal form B is basically as shown in Figure 7.

The present disclosure also provides a use of the crystal form A or/and the crystal form B in the manufacture of a porcupine inhibitor medicament.

The present disclosure also provides a use of the crystal form A or/and the crystal form B in the manufacture of a medicament for treating pancreatic cancer, colorectal cancer, and gastric cancer.

The present disclosure also provides a use of the compound or the pharmaceutically acceptable salt thereof or/and the crystal form A of compound 2 or/and the crystal form B of compound 2 in the manufacture of a porcupine inhibitor medicament.

The present disclosure also provides a use of the compound or the pharmaceutically acceptable salt thereof or/and the crystal form A of compound 2 or/and the crystal form B of compound 2 in the manufacture of a medicament for treating pancreatic cancer, colorectal cancer, and gastric cancer.

The present disclosure also provides a biological experimental test method for the above compounds:

### Experimental test method 1: In vivo drug efficacy experiment in Capan-2 nude mouse xenograft

Capan-2 cells are cultured in McCoy's 5A medium, containing 10% fetal bovine serum (FBS), in a 37°C incubator with 5% CO₂. The tumor cells are collected during the logarithmic growth phase after they have passaged and grown to an appropriate concentration. The cells are resuspended in McCoy's 5A medium and adjusted to a cell suspension concentration of 2 × 10⁷/mL for inoculation.

The establishment of human pancreatic cancer Capan-2 xenografts. Cells are collected and adjusted to a concentration of 2 × 10⁷ cells/mL (resuspended in McCoy's 5A medium to form a cell suspension containing 50% Matrigel). Under sterile conditions, 0.1 mL of tumor cells is subcutaneously injected into the right side of the mouse's back, with each mouse being inoculated with 2 × 10⁶ cells. Once the tumor has grown to a certain size, a digital caliper is used to measure the length (a) and width (b) of the tumor, and the tumor volume (TV) is calculated. The formula for calculating the tumor volume is: TV = a × b² / 2.

Capan-2 tumor cells are inoculated and allowed to grow until the tumor size reaches approximately 150 cubic millimeters. The animals are then divided into groups, with 6 animals per group. The day of grouping is considered as day 0, and administration is carried out on the day of grouping. During the experiment, the weight and tumor size of the animals are measured twice a week, and the clinical symptoms of the animals are observed and recorded daily. Each administration is based on the most recent weight measurement of the animal.

The evaluation index for anti-tumor activity is the relative tumor proliferation rate T/C (%). If T/C (%) > 40%, it is considered ineffective. If T/C (%) ≤ 40%, and after statistical processing, P < 0.05, it is considered effective. The formula for calculating T/C (%) is: T/C (%) = (T_{RTV} / C_{RTV}) × 100%. T_{RTV} is the relative tumor volume of the treatment group, and C_{RTV} is the relative tumor volume of the negative control group. The tumor growth inhibition rate TGI (%)^{b} = (average tumor volume or weight of the negative control group - average tumor volume or weight of the administration group) / average tumor volume or weight of the negative control group × 100%. TGI (%)^{a} = [(1 - (average tumor volume of a certain administration group at the time of measurement - average tumor volume of the administration group at the start of administration)) / (average tumor volume of the solvent control group at the time of measurement - average tumor volume of the solvent control group at the start of treatment)] * 100%.

### Technical effect

The compound of the present disclosure has a good inhibitory effect on porcupine protein, can effectively regulate the Wnt/β-Catenin pathway signal, and has a good growth inhibitory effect on tumors with excessive activation of the Wnt/β-Catenin signaling pathway, as well as good pharmacokinetic properties.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

It must be noted that the singular forms "a", "an", "the", and similar terms used in the contents of the present disclosure (especially the contents of the appended claims) as used herein and in the appended claims should be interpreted as including both the singular and the plural, unless otherwise explicitly stated in the context or clearly contrary to the present specification. Therefore, for example, reference to "the compound" includes reference to one or more than one compound; and so on.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereoisomers, (D)-isomers, (L)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 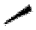 ) and a wedged dashed bond ( 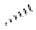 ), and the relative configuration of a stereogenic center is represented by a straight solid bond ( 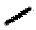 ) and a straight dashed bond ( 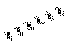 ), a wave line ( 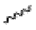 ) is used to represent a wedged solid bond ( 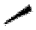 ) or a wedged dashed bond ( 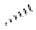 ), or the wave line ( 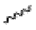 ) is used to represent a straight solid bond ( 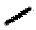 )or a straight dashed bond ( 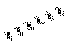 ).

The compounds of the present disclosure may exist in specific forms. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes interconversions carried out by the reorganization of some bonding electrons. A specific example of keto-enol tautomerization is the tautomerism between two tautomers of pentane-2, 4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (R)- and (S)-isomers, and *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine). The compound of the present disclosure may contain an unnatural proportion of atomic isotope at one or more atoms that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 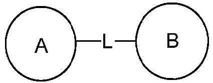 is -M-W-, then -M-W- can link ring A and ring B to form 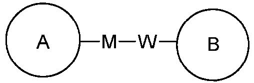 in the direction same as left-to-right reading order, and form 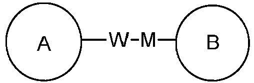 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( 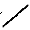 ), a straight dashed bond ( 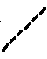 ), or a wavy line ( 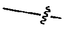 ). For example, the straight solid bond in -OCH₃ means that it is linked to other groups through the oxygen atom in the group; the straight dashed bond in 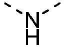 means that it is linked to other groups through the two ends of the nitrogen atom in the group; the wave lines in 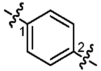 means that the phenyl group is linked to other groups through carbon atoms at position 1 and position 2; 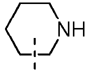 means that it can be linked to other groups through any linkable sites on the piperidinyl by one chemical bond, including at least four types of linkage, including 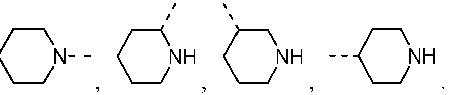 Even though the H atom is drawn on the -N-, 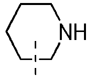 still includes the linkage of 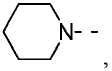 merely when one chemical bond was connected, the H of this site will be reduced by one to the corresponding monovalent piperidinyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including n-propyl and isopropyl), etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, C₂ alkoxy, etc. Examples of C₁₋₃ alkoxy include, but are not limited to, methoxy, ethoxy, propoxy (including n-propoxy and isopropoxy), etc.

Unless otherwise specified, the term "C₁₋₃ alkylamino" refers to an alkyl group containing 1 to 3 carbon atoms that are connected to the rest of the molecule through an ammonia atom. The C₁₋₃ alkylamino includes C₁₋₂, C₃, C₂ alkylamino, etc. Examples of C₁₋₃ alkylamino include, but are not limited to, -NHCH₃, -N(CH₃)₂, -NHCH₂CH₃, - N(CH₃)CH₂CH₃, -NHCH₂CH₂CH₃, -NHCH₂(CH₃)₂, etc.

Unless otherwise specified, the terms "9- to 10-membered heteroaryl ring" and "9- to 10-membered heteroaryl" in the present disclosure can be used interchangeably, and the term "9- to 10-membered heteroaryl" refers to a cyclic group consisting of 9 to 10 ring atoms with a conjugated π-electron system, wherein 1, 2, 3, or 4 ring atoms are heteroatoms independently selected from O, S, and N, and the rest are carbon atoms. It can be a monocyclic, condensed bicyclic, or condensed tricyclic system, wherein each ring is aromatic. Here, the nitrogen atom is optionally quaternized, and nitrogen and sulfur heteroatoms can be optionally oxidized (i.e., NO and S(O)ₚ, wherein p is 1 or 2). The 5- to 10-membered heteroaryl can be linked to the rest of the molecule through a heteroatom or a carbon atom. The 9- to 10-membered heteroaryl includes 9-membered, 10-membered heteroaryl, etc. Examples of the 9- to 10-membered heteroaryl include but are not limited to benzothiazolyl (including 5-benzothiazolyl, etc.), purinyl, benzimidazolyl (including 2-benzimidazolyl, etc.), benzoxazolyl, indolyl (including 5-indolyl, etc.), isoquinolyl (including 1-isoquinolyl and 5-isoquinolyl, etc.), quinoxalinyl (including 2-quinoxalinyl and 5-quinoxalinyl, etc.), or quinolinyl (including 3-quinoxalinyl and 6- quinolinyl, etc.).

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, etc.

The term "leaving group" refers to a functional group or atom which can be replaced by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonate; acyloxy, such as acetoxy, trifluoroacetoxy.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for blocking the side reaction on hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

Throughout this specification, references to "an embodiment" or "embodiments" or "in another embodiment" or "in some embodiments" mean that at least one embodiment includes a specific reference element, structure, or feature related to what was described in that embodiment Thus, appearances of the phrases "in an embodiment" or "in embodiments" or "in another embodiment" or "in some embodiments" in various places throughout this specification are not necessarily all referring to the same embodiment. Furthermore, specific elements, structures, or features may be combined in one or more than one embodiment in any suitable manner.

Differential scanning calorimetry (DSC) of the crystal forms described in the present disclosure is subject to experimental error and is slightly affected by the degree of dryness of the sample. From one machine to another and from one sample to another, the position and peak value of the endothermic peak may be slightly different, and the values of the experimental error or the difference may be less than or equal to 10°C, or less than or equal to 9°C, or less than or equal to 8°C, or less than or equal to 7°C, or less than or equal to 6°C, or less than or equal to 5°C, or less than or equal to 4°C, or less than or equal to 3°C, or less than or equal to 2°C, or less than or equal to 1°C, so the peak position or peak value of the DSC endothermic peak cannot be regarded as absolute.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The present disclosure is described in detail by the examples below.

The solvent used in the present disclosure is commercially available.

In the present disclosure, the following abbreviations are adopted: DMF stands for N,N-dimethylformamide; K₂CO₃ stands for potassium carbonate; Cs₂CO₃ stands for cesium carbonate; EtOAc stands for ethyl acetate; EA stands for ethyl acetate; THF stands for tetrahydrofuran; MeOH stands for methanol; DCM stands for dichloromethane; DMSO stands for dimethyl sulfoxide; PE stands for petroleum ether; EtOH stands for ethanol; ACN stands for acetonitrile; TFA stands for trifluoroacetic acid; FA stands for formic acid; NH₃·H₂O stands for ammonia water; TEA stands for triethylamine; DIPEA stands for *N,N-*diisopropylethylamine; Boc₂O stands for *di-tert-butyl* dicarbonate; Boc stands for *tert-*butoxycarbonyl, which is a protecting group for amino; LCMS stands for liquid chromatography-mass spectrometry; HPLC stands for high performance liquid chromatography; TLC stands for thin-layer chromatography; SFC stands for supercritical fluid chromatography. g stands for gram; mg stands for milligram; µL stands for microliter; mL stands for milliliter; mol stands for mole; mmol stands for millimole; µmol stands for micromole; M stands for mole/liter; mM stands for millimole/liter; µM stands for micromole/liter; nM stands for nanomole/liter. Me stands for methyl; Boc stands for *tert-*butoxycarbonyl; DMSO-*d₆* stands for deuterated dimethyl sulfoxide; CD₃OD-*d*₄ stands for deuterated methanol; CDCl₃ stands for deuterated chloroform.

The compounds of the present disclosure are named according to the conventional naming principles in the art or by ChemDraw^{®} software, and the commercially available compounds use the supplier catalog names.

**The test parameters of the X-ray powder diffractometer (XRPD) method of the present disclosure are shown in Table 3.**

**Table 3 XRPD test parameters**

| Parameters | Set value |
|---|---|
| Model | Empyrean |
| X-ray | Cu, Kα, |
| | Kα1 (Å):1.54060, |
| | Kα2 (Å):1.54443, |
| | Kα2 / Kα1 intensity ratio: 0.50 |
| X-ray tube setting | 45 kV, 40 mA |
| Divergent slit | 1/8° |
| Scanning mode | Continuous |
| Scanning range (°2θ) | 3 to 40 |
| Scanning time per step (s) | 46.7 |
| Scanning step size (°2θ) | 0.0263 |
| Test time (min) | About 5 min |

**The test parameters of the differential scanning calorimeter (DSC) method of the present disclosure are shown in Table 4.**

**Table 4 DSC test parameters**

| DSC test parameters | |
|---|---|
| Instrument model | TA Discovery DSC 2500 |
| Method | Linear heating |
| Sample tray | Aluminum tray, gland |
| Temperature range | 25 to 350°C |
| Scanning rate (°C/min) | 10 |
| Protective gas | Nitrogen |

**The test parameters of the thermal gravimetric analyzer (TGA) method of the present disclosure are shown in Table 5.**

**Table 5 TGA test parameters**

| TGA test parameters | |
|---|---|
| Instrument model | TA Discovery TGA 5500 |
| Method | Linear heating |
| Instrument model | TA Discovery TGA 5500 |
| Sample tray | Aluminum tray, open lid |
| Temperature range | Room temperature to 350°C |
| Scanning rate (°C/min) | 10 |
| Protective gas | Nitrogen |

**The test parameters of the dynamic vapor sorption (DVS) method of the present disclosure are shown in Table 6.**

**Table 6 DVS test parameters**

| **Parameters** | **Set value** |
|---|---|
| Instrument manufacturer model | SMS DVS intrinsic dynamic vapor sorption instrument |
| Test conditions | 10 to 30 mg |
| Temperature | 25°C |
| Protective gas and flow rate | N₂, 200 mL/min |
| Equilibrium | dm/dt: 0.002%/min |
| Minimum dm/dt equilibration time | 10 min |
| Maximum equilibration time | 180 min |
| RH range | 30%RH-95%RH-0%RH-95%RH |
| RH gradient | 10% (90%RH-0%RH-90%RH) |
| | 5% (95%RH-90%RH, 90%RH-95%RH) |

### BRIEF DESCRIPTION OF THE DRAWINGS

Figure 1 is the XRPD pattern of Cu-Kα radiation of crystal form A of compound 2;
Figure 2 is the DSC pattern of crystal form A of compound 2;
Figure 3 is the TGA pattern of crystal form A of compound 2;
Figure 4 is the DVS pattern of crystal form A of compound 2;
Figure 5 is the XRPD pattern of Cu-Kα radiation of crystal form B of compound 2 (not part of the present invention);
Figure 6 is the DSC pattern of crystal form B of compound 2 (not part of the present invention);
Figure 7 is the TGA pattern of crystal form B of compound 2 (not part of the present invention).

### DETAILED DESCRIPTION OF THE PREFERRED EMBODIMENT

The present disclosure is described in detail by the examples below.

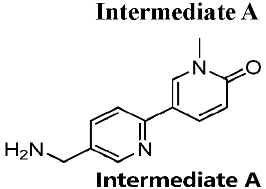

### Synthetic route:

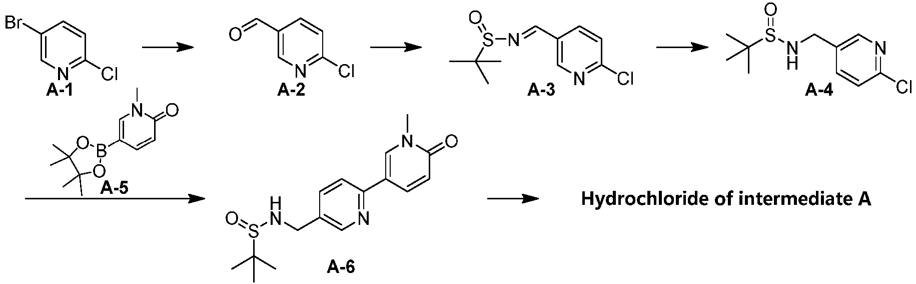

### Step 1

Compound A-1 (15 g, 77.95 mmol) was dissolved in tetrahydrofuran (150 mL), then N-formylmorpholine (11.67 g, 101.33 mmol) and isopropyl magnesium chloride-lithium chloride (1.3 M, 89.94 mL) were added to the reaction mixture. Gas displacement was carried out and the reaction mixture was stirred at 25°C for 2 hours under a nitrogen atmosphere. After the reaction was completed, the reaction mixture was diluted with water (300 mL) and extracted with ethyl acetate (100 mL × 3). The combined organic phases were washed with saturated sodium chloride solution (200 mL), dried over anhydrous sodium sulfate, filtered, and concentrated under reduced pressure to obtain crude product **A-2.**

### Step 2

Compound **A-2** (11 g, 77.71 mmol) was dissolved in tetrahydrofuran (150 mL), and then tert-butylsulfenamide (18.84 g, 155.42 mmol), sodium sulfate (5.52 g, 38.85 mmol), and copper sulfate (37.21 g, 233.12 mmol) were added to the reaction mixture. The reaction mixture was stirred at 80°C for 12 hours. After the reaction was completed, the reaction mixture was filtered, and the filtrate was diluted with water (200 mL) and extracted with ethyl acetate (150 mL × 3). The combined organic phases were washed with saturated sodium chloride solution (300 mL), dried over anhydrous sodium sulfate, filtered, and concentrated under reduced pressure to obtain crude product **A-3.** MS-ESI calculated for [M+H]⁺ 245, found 245.

### Step 3

Compound **A-3** (19 g, 77.63 mmol) was dissolved in tetrahydrofuran (200 mL), and sodium borohydride (1.81 g, 47.85 mmol) was added to the reaction mixture. The reaction mixture was stirred at 25°C for 2 hours. After the reaction was completed, the reaction mixture was diluted with water (350 mL) and extracted with ethyl acetate (150 mL × 3). The combined organic phases were washed with saturated sodium chloride solution (300 mL), dried over anhydrous sodium sulfate, filtered, concentrated under reduced pressure, and subjected to silica gel column chromatography (petroleum ether: ethyl acetate = 100:1 to 1:1) to obtain compound **A-4.** MS-ESI calculated for [M+H]⁺ 247, found 247.

### Step 4

Compound **A-4** (1.38 g, 5.61 mmol) was dissolved in dioxane (20 mL) and water (10 mL). Compound **A-5** (1.45 g, 6.17 mmol), potassium carbonate (1.55 g, 11.22 mmol), and [1,1'-bis(diphenylphosphino)ferrocene]dichloropalladium(II) (205.14 mg, 280.45 µmol) were added to the reaction mixture. The reaction mixture was stirred at 80°C for 4 hours. After the reaction was completed, the reaction mixture was filtered, concentrated under reduced pressure, and subjected to silica gel column chromatography (petroleum ether: ethyl acetate = 10/1 to 1/1) to obtain compound **A-6.** MS-ESI calculated for [M+H]⁺ 320, found 320.

### Step 5

Compound A-6 (2.8 g, 8.77 mmol) was dissolved in methanol (20 mL), and hydrogen chloride/ethyl acetate (4 M, 10.96 mL) was added to the reaction mixture. The reaction mixture was stirred at 25°C for 4 hours. After the reaction was completed, the reaction mixture was filtered, and the filter cake was dried under vacuum to obtain the **hydrochloride of intermediate A.** MS-ESI calculated for [M+H]⁺ 216, found 216.

### Example 1

### Synthetic route:

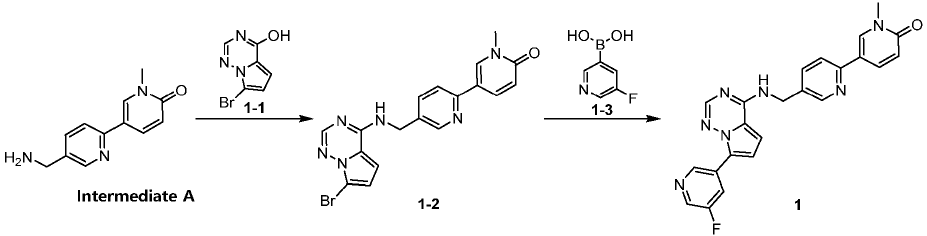

### Step 1

The **hydrochloride of intermediate A** (200 mg) and compound 1-1 (118.67 mg, 554.47 µmol) were dissolved in N,N-dimethylformamide (3 mL). *N,N-*dimethylisopropylamine (636.99 mg, 4.93 mmol) and benzotriazol-1-yloxytris(dimethylamino)phosphonium hexafluorophosphate (35.97 mg, 985.73 µmol) were added to the reaction mixture. The reaction mixture was stirred at 50°C for 12 hours. After the reaction was completed, the reaction mixture was diluted with water (5 mL) and extracted with ethyl acetate (10 mL × 3). The combined organic phases were washed with saturated sodium chloride solution (10 mL × 2), dried over anhydrous sodium sulfate, filtered, and the filtrate was concentrated under reduced pressure and subjected to silica gel column chromatography (eluent: dichloromethane/methanol, 1/0 to 10/1, V/V) to obtain crude compound **1-2.** MS-ESI calculated for [M+H]⁺ 412, found 412.

### Step 2

Compound **1-2** (80 mg, 194.53 µmol), compound **1-3** (54.82 mg, 389.05 µmol), and potassium phosphate (123.87 mg, 583.58 µmol) were dissolved in tetrahydrofuran (2 mL) and water (0.5 mL). Nitrogen replacement was carried out three times for the reaction mixture, then 1,1'-bis(di-tert-butylphosphino)ferrocene palladium dichloride (25.36 mg, 38.91 µmol) was added under a nitrogen atmosphere, and stirred at 70°C under a nitrogen atmosphere for 12 hours. After the reaction was completed, the reaction mixture was filtered, and the filtrate was concentrated under reduced pressure, then diluted with water (5 mL), and extracted with a mixture of dichloromethane and methanol (10:1, V/V, 10 mL × 3). The combined organic phases were washed with saturated sodium chloride solution (10 mL × 2), dried over anhydrous sodium sulfate, filtered, and concentrated under reduced pressure to obtain crude product. The crude product was first purified by thin-layer chromatography (developing solvent: dichloromethane/methanol 10/1, V/V) to obtain a crude product, which was then subjected to high performance liquid chromatography (chromatographic column: Phenomene × luna C18 150 × 40 mm × 15 µm; mobile phase: mobile phase A: 0.225% formic acid aqueous solution by volume; mobile phase B: acetonitrile; B%: 28% to 55%) to obtain compound 1.

MS-ESI calculated for [M+H]⁺ 428, found 428. ¹H NMR (400 MHz, DMSO-*d*₆) δ ppm 9.16 (t, *J =* 1.6 Hz, 1 H), 9.01 (t, *J =* 5.8 Hz, 1 H), 8.61 (d, *J =* 1.4 Hz, 1 H) 8.45 - 8.54 (m, 3 H), 8.10 - 8.18 (m, 2 H), 7.75 - 7.85 (m, 2 H), 7.33 (d, *J* = 4.6 Hz, 1 H), 7.12 (d, *J =* 4.6 Hz, 1 H), 6.49 (d, *J =* 9.4 Hz, 1 H), 4.80 (d, *J =* 5.8 Hz, 2 H), 3.53 (s, 3 H).

### Example 2

### Synthetic route:

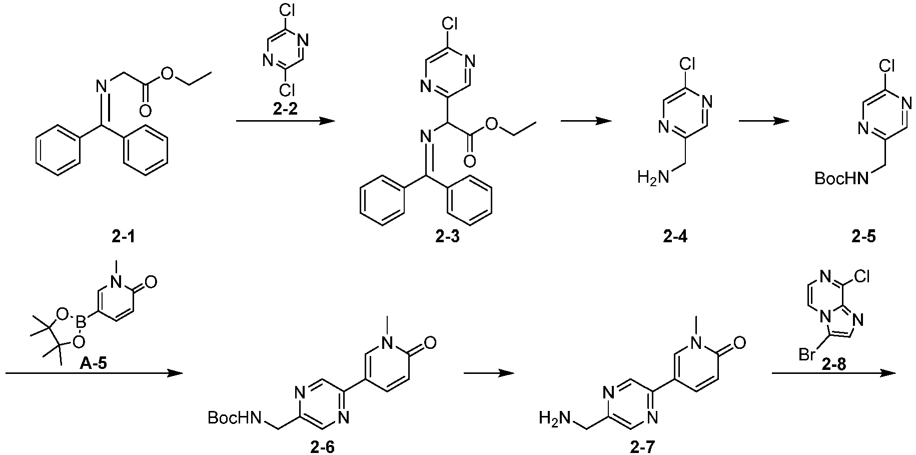

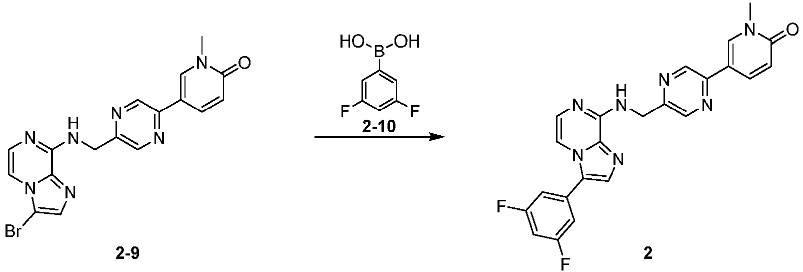

### Step 1

Compound 2-1 (7.9 g, 29.53 mmol), compound **2-2** (4.4 g, 29.53 mmol), and cesium carbonate (9.62 g, 29.53 mmol) were dissolved in *N,N*-dimethylformamide (44 mL). Nitrogen replacement was carried out three times for the reaction mixture, and the reaction mixture was stirred at 20°C for 21 hours under a nitrogen atmosphere. After the reaction was completed, the reaction mixture was diluted with water (50 mL) and extracted with ethyl acetate (100 mL × 3). The combined organic phases were washed with saturated sodium chloride solution (50 mL × 2), dried over anhydrous sodium sulfate, filtered, concentrated under reduced pressure, and subjected to silica gel column chromatography (eluent: petroleum ether/ethyl acetate, 1/0 to 20/1, V/V) to obtain crude product **2-3.** MS-ESI calculated for [M+H]⁺ 380, found 380.

### Step 2

Compound 2-3 (10 g, 26.33 mmol) was dissolved in water (9.1 mL) and toluene (27 mL), then hydrochloric acid (2.67 g, 26.33 mmol, purity: 36%) was added thereto, and the mixture was reacted at 25°C for 21 hours. After the reaction was completed, the reaction mixture was extracted with toluene (20 mL × 3), and the aqueous phases were combined. Concentrated hydrochloric acid (2.36 mL) was added to the aqueous phase, and the resulting mixture was reacted at 60°C for 20 hours. After the reaction was completed, the reaction mixture was concentrated under reduced pressure to obtain the **hydrochloride of compound 2-4.** MS-ESI calculated for [M+H]⁺ 144, found 144.

### Step 3

The **hydrochloride of compound 2-4** (3.8 g) and sodium bicarbonate (22.23 g, 264.67 mmol) were dissolved in dichloromethane (35 mL) and acetonitrile (35 mL), then *di-tert-butyl* dicarbonate (8.66 g, 39.70 mmol) was added thereto, and the mixture was reacted at 25°C for 16 hours. After the reaction was completed, the reaction mixture was diluted with water (100 mL) and extracted with ethyl acetate (100 mL × 3). The combined organic phases were washed with saturated sodium chloride solution (50 mL × 2), dried over anhydrous sodium sulfate, filtered, concentrated under reduced pressure, and subjected to silica gel column chromatography (eluent: petroleum ether/ethyl acetate, 1/0 to 10/1, V/V) to obtain crude product **2-5.** MS-ESI calculated for [M+H]⁺ 244, found 244.

### Step 4

Compound **2-5** (3 g, 12.31 mmol), compound **A-5** (3.47 g, 14.77 mmol), [1,1'-bis(diphenylphosphino)ferrocene]dichloropalladium(II) (450.39 mg, 615.54 µmol), and potassium carbonate (3.40 g, 24.62 mmol) were dissolved in water (3 mL) and dioxane (30 mL). Nitrogen replacement was carried out three times for the reaction mixture, and the reaction mixture was stirred at 80°C for 12 hours under a nitrogen atmosphere. After the reaction was completed, the reaction mixture was diluted with water (50 mL) and extracted with ethyl acetate (100 mL × 2). The combined organic phases were washed with saturated sodium chloride solution (50 mL × 2), dried over anhydrous sodium sulfate, filtered, concentrated under reduced pressure and subjected to silica gel column chromatography (eluent: dichloromethane/methanol, 1/0 to 50/1, V/V) to obtain crude product **2-6.** MS-ESI calculated for [M+H]⁺ 317, found 317.

### Step 5

Compound **2-6** (5.64 g, 17.83 mmol) was dissolved in methanol (50 mL), then hydrogen chloride/ethyl acetate (44.57 mL, 178.3 mmol, 4M) was added thereto, and the reaction mixture was reacted at 25°C for 12 hours. After the reaction was completed, the reaction mixture was concentrated under reduced pressure to obtain a crude product, and a mixture of ethyl acetate and methanol (55 mL, 10/1, V/V) was added to the crude product. The reaction mixture was stirred at 25°C for 1 hour, and then filtered to obtain a filter cake. The filter cake was concentrated under reduced pressure to obtain the **hydrochloride of compound 2-7.** MS-ESI calculated for [M+H]⁺ 217, found 217.

### Step 6

The **hydrochloride of compound 2-7** (1 g) was dissolved in *N*-methylpyrrolidone (10 mL), then N,N-dimethylisopropylamine (1.8 g, 13.96 mmol) and compound **2-8** (584.11 mg, 2.51 mmol) were added thereto, and the reaction mixture was reacted at 130°C for 16 hours. After the reaction was completed, the reaction mixture was diluted with water (50 mL), filtered to obtain a filter cake, and the filter cake was concentrated under reduced pressure to obtain compound **2-9.** MS-ESI calculated for [M+H]⁺ 413, found 413.

Step 7Compound **2-9** (400 mg, 970.3 µmol), compound **2-10** (229.83 mg, 1.46 mmol), [1,1'-bis(diphenylphosphino)ferrocene]dichloropalladium(II) (35.5 mg, 48.52 µmol), and potassium carbonate (268.2 mg, 1.94 mmol) were dissolved in dioxane (4 mL) and water (0.8 mL). Nitrogen replacement was carried out three times for the reaction mixture, and the reaction mixture was stirred at 80°C for 12 hours under a nitrogen atmosphere. After the reaction was completed, the reaction mixture was then diluted with water (20 mL) and extracted with ethyl acetate (50 mL × 3). The combined organic phases were washed with saturated sodium chloride solution (20 mL × 2), dried over anhydrous sodium sulfate, filtered, and concentrated under reduced pressure to obtain a crude product. The crude product was first subjected to silica gel column chromatography (eluent: dichloromethane/methanol, 1/0 to 20/1, V/V) to obtain a crude product, which was then subjected to high performance liquid chromatography (chromatographic column: Phenomene × Gemini -N × C18 75 × 30 mm ×15 µm; mobile phase: mobile phase A: 0.05% ammonia solution by volume; mobile phase B: acetonitrile; B%: 18% to 48%) to obtain compound **2.**

MS-ESI calculated for [M+H]⁺ 446, found 446. ¹H NMR (400 MHz, DMSO-*d*₆) δ ppm 9.03 (d, *J =* 1.4 Hz, 1 H), 8.59 (m, 2 H), 8.28 (m, 1 H) 8.16 (m, 1 H), 7.85 - 7.95 (m, 2 H), 7.47 (m, 2 H), 7.30 - 7.40 (m, 2 H), 6.53 (d, *J =* 9.6 Hz, 1 H), 4.85 (d, *J =* 6.0 Hz, 2 H), 3.54 (s, 3 H).

### Example 3

### Synthetic route:

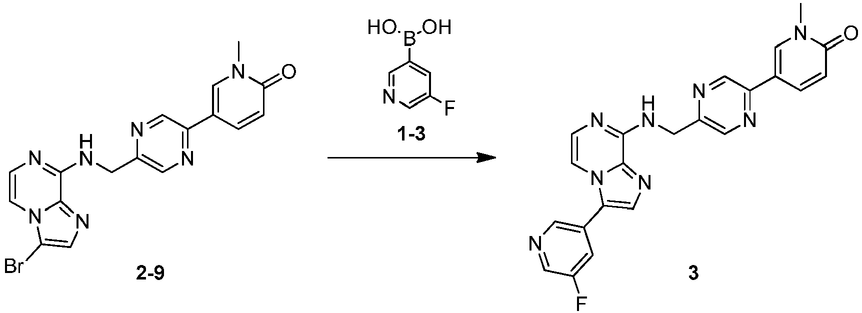

Compound 2-9 (400 mg, 970.3 µmol), compound **1-3** (205.08 mg, 1.46 mmol), [1,1'-bis(diphenylphosphino)ferrocene]dichloropalladium(II) (25.5 mg, 48.52 µmol), and potassium carbonate (268.2 mg, 1.94 mmol) were dissolved in dioxane (4 mL) and water (0.8 mL). Nitrogen replacement was carried out three times for the reaction mixture, 1,1'-bis(di-tert-butylphosphino)ferrocene palladium dichloride (25.36 mg, 38.91 µmol) was added under a nitrogen atmosphere, and stirred at 80°C for 12 hours under a nitrogen atmosphere. After the reaction was completed, the reaction mixture was filtered, and the filtrate was concentrated under reduced pressure, then diluted with water (20 mL) and extracted with ethyl acetate (50 mL × 3). The combined organic phases were washed with saturated sodium chloride solution (20 mL × 2), dried over anhydrous sodium sulfate, filtered, and concentrated under reduced pressure to obtain a crude product. The crude product was first purified by silica gel column chromatography (eluent: dichloromethane/methanol, 1/0 to 20/1, V/V) to obtain a crude product, which was then purified by high performance liquid chromatography (chromatographic column: Waters × bridge C18 150 × 50 mm × 10 µm; mobile phase: mobile phase A: 10 mM ammonium bicarbonate aqueous solution; mobile phase B: acetonitrile; B%: 14% to 44%) to obtain compound 3.

MS-ESI calculated for [M+H]⁺ 429, found 429. ¹H NMR (400 MHz, DMSO-*d*₆) δ ppm 9.03 (d, *J =* 1.4 Hz, 1 H), 8.79 (s, 1 H), 8.67 (d, *J =* 2.6 Hz, 1 H), 8.60 (m, 2 H), 8.31 (m, 1 H), 8.09 - 8.20 (m, 2 H), 7.97 (s, 1 H), 7.91 (d, *J =* 4.8 Hz, 1 H), 7.34 (d, *J =* 4.8 Hz, 1 H), 6.53 (d, *J=* 9.6 Hz, 1 H), 4.85 (d, *J =* 5.8 Hz, 2 H), 3.54 (s, 3 H).

### Example 4

### Synthetic route:

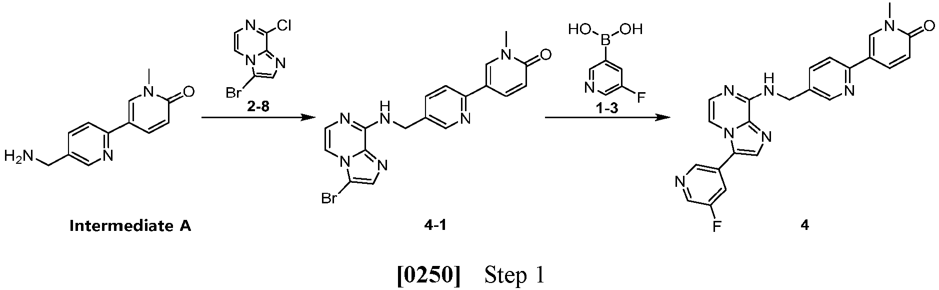

The **hydrochloride of compound intermediate A** (500 mg) and compound **2-8** (539.99 mg, 2.32 mmol) were dissolved in N-methylpyrrolidone (15 mL), and *N,N-*dimethylisopropylamine (300.21 mg, 2.32 mmol) was added to the reaction mixture. The reaction mixture was stirred at 130°C for 16 hours. After the reaction was completed, the reaction mixture was diluted with water (100 mL) and extracted with ethyl acetate (100 mL × 3). The combined organic phases were washed with saturated sodium chloride solution (100 mL × 3), dried over anhydrous sodium sulfate, filtered, concentrated under reduced pressure, and subjected to silica gel column chromatography (petroleum ether: ethyl acetate: ethanol = 4:3:1) to obtain crude product **4-1.** MS-ESI calculated for [M+H]⁺ 412, found 412.

### Step 2

Compound **4-1** (955 mg, 2.32 mmol) and compound **1-3** (425.37 mg, 3.02 mmol) were dissolved in dioxane (10 mL) and water (2 mL), then potassium carbonate (962.81 mg, 6.97 mmol) and [1,1'-bis(diphenylphosphino)ferrocene]dichloropalladium(II) (169.91 mg, 232.22 µmol) were added to the reaction mixture. Gas displacement was carried out and the reaction mixture was stirred at 110°C for 16 hours under a nitrogen atmosphere. After the reaction was completed, the reaction mixture was filtered, concentrated under reduced pressure, and subjected to high performance liquid chromatography (chromatographic column: Phenomene × luna C18 150 × 40 mm × 15 µm; mobile phase: mobile phase A: 0.225% formic acid aqueous solution by volume; mobile phase B: acetonitrile; B%: 15% to 45%) to obtain compound **4.**

MS-ESI calculated for [M+H]⁺ 428, found 428. ¹H NMR (400 MHz, DMSO-*d*₆) δ ppm 8.77 (t, *J =* 1.6 Hz, 1 H), 8.66 (d, *J =* 2.8 Hz, 1 H), 8.27 (s, 1 H), 8.07 (d, *J =* 2.8 Hz, 1 H), 7.9 (s, 1 H), 7.86 (d, *J =* 4.8Hz, 1 H), 7.79 (d, *J* = 2.6 Hz, 1 H), 7.46 - 7.51 (m, 2 H), 7.38 - 7.43 (m, 2 H), 7.4 (d, *J =* 4.8 Hz, 1 H), 6.46 (d, *J =* 9.4 Hz, 1 H), 4.7 (d, *J =* 6.4 Hz, 2 H), 3.49 (s, 3 H).

### Example 5

### Synthetic route:

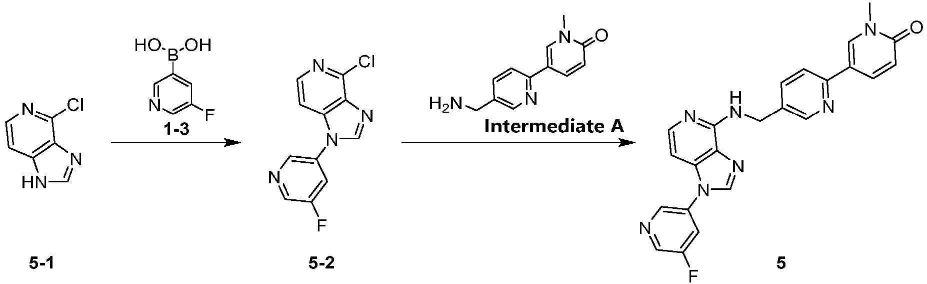

### Step 1

Compound 5-1 (2 g, 13.02 mmol) was dissolved in N,N-dimethylformamide (10 mL). Compound 1-3 (2.39 g, 16.93 mmol), copper acetate (4.73 g, 26.05 mmol), and pyridine (3.09 g, 39.07 mmol) were added to the reaction mixture. Gas displacement was carried out and the reaction mixture was stirred at 40°C for 12 hours under an oxygen atmosphere. After the reaction was completed, the reaction mixture was added with water (100 mL) and extracted with dichloromethane (100 mL). The combined organic phases were washed with saturated sodium chloride solution (25 mL × 2), dried over anhydrous sodium sulfate, filtered, concentrated under reduced pressure, and subjected to silica gel column chromatography (petroleum ether: ethyl acetate = 10/1 to 2/1) to obtain compound **5-2.** MS-ESI calculated for [M+H]⁺ 249, found 249.

### Step 2

Compound 5-2 (330 mg, 1.33 mmol) and the **hydrochloride of intermediate A** (428.52 mg) were dissolved in N-methylpyrrolidone (5 mL), and N,N-dimethylisopropylamine (280.94 mg, 2.03 mmol) was added to the reaction mixture. The reaction mixture was stirred at 130°C for 3 hours. After the reaction was completed, the reaction mixture was diluted with water (20 mL) and extracted with ethyl acetate (20 mL). The organic phase was washed with saturated sodium chloride solution (10 mL × 2) and subjected to high performance liquid chromatography (chromatographic column: Phenomene × luna C18 75 × 30 mm × 3 µm; mobile phase: mobile phase A: 0.225% formic acid aqueous solution by volume; mobile phase B: acetonitrile; B%: 1% to 30%) to obtain compound **5.**

MS-ESI calculated for [M+H]⁺ 428, found 428. ¹H NMR (400 MHz, DMSO-*d*₆) δ ppm 8.84 (d, *J* = 1.6 Hz, 1 H), 8.75 (d, *J =* 2.4 Hz, 1 H), 8.59 (d, *J =* 1.6 Hz, 1 H), 8.56 (s, 1 H), 8.48 (d, *J =* 2.4 Hz, 1 H), 8.24 (m, 1 H), 8.12 (m, 1 H), 7.84 (d, *J =* 6.0 Hz, 1 H), 7.79 (m, 1 H), 7.73 (s, 1 H), 7.66 (t, *J =* 6.2 Hz, 1 H), 6.92 (d, *J = 5.8* Hz, 1 H), 6.48 (d, *J =* 9.6 Hz, 1 H), 4.74 (d, *J =* 6.0 Hz, 2 H), 3.52 (s, 3 H).

### Example 6

### Synthetic route:

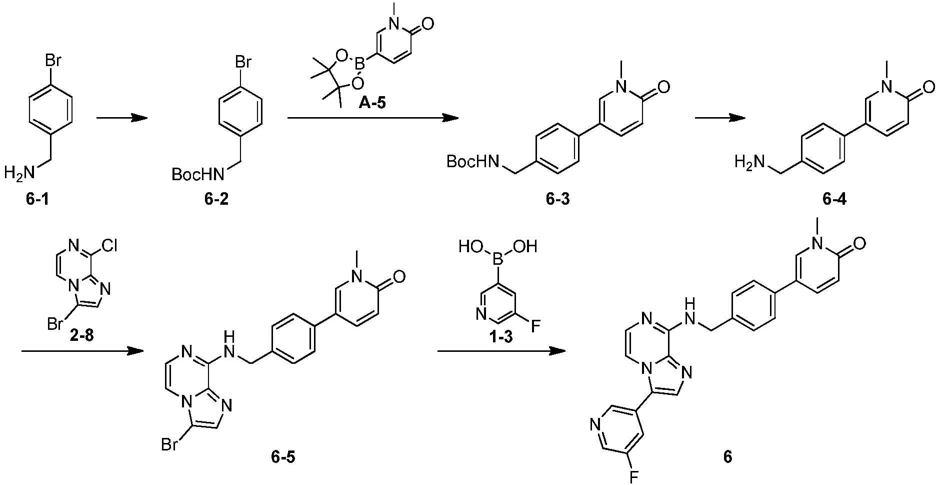

### Step 1

Compound 6-1 (5 g, 26.87 mmol) was dissolved in dichloromethane (50 mL), then *di-*tert-butylmethyl dicarbonate (7.04 g, 32.25 mmol) and N,N-dimethylisopropylamine (10.42 g, 80.62 mmol) were added to the reaction mixture. Gas displacement was carried out and the reaction mixture was stirred at 25°C for 12 hours under a nitrogen atmosphere. After the reaction was completed, the reaction mixture was added with water (100 mL) and extracted with dichloromethane (200 mL). The combined organic phases were washed with saturated sodium chloride solution (50 mL × 2), dried over anhydrous sodium sulfate, filtered, and concentrated under reduced pressure to obtain compound **6-2.** MS-ESI calculated for [M+H]⁺ 287, found 287.

### Step 2

Compound **6-2** (2 g, 6.99 mmol) and compound **A-5** (2.05 g, 8.74 mmol) were dissolved in dioxane (30 mL) and water (3 mL), then potassium carbonate (2.90 g, 20.97 mmol) and [1,1'-bis(diphenylphosphino)ferrocene]dichloropalladium(II) (511.39 mg, 698.90 µmol) were added to the reaction mixture. The reaction mixture was stirred at 80°C for 12 hours. After the reaction was completed, the reaction mixture was filtered, concentrated under reduced pressure, and subjected to silica gel column chromatography (petroleum ether/ethyl acetate = 5/1 to 0/1) to obtain compound **6-3.** MS-ESI calculated for [M+H]⁺ 315, found 315.

### Step 3

Compound **6-3** (2 g, 6.36 mmol) was dissolved in ethyl acetate (40 mL), and hydrogen chloride/ethyl acetate (4 M, 7.95 mL) was added to the reaction mixture. Gas displacement was carried out and the reaction mixture was stirred at 25°C for 12 hours under a nitrogen atmosphere. After the reaction was completed, the reaction mixture was filtered, and the filter cake was dried under vacuum to obtain the **hydrochloride of compound 6-4.**

### Step 4

The **hydrochloride of compound 6-4** (2 g) and compound **2-8** (1.84 g, 7.93 mmol) were dissolved in N-methylpyrrolidone (20 mL), and N,N-dimethylisopropylamine (6.03 g, 46.67 mmol) was added to the reaction mixture. Gas displacement was carried out and the reaction mixture was stirred at 130°C for 12 hours under a nitrogen atmosphere. After the reaction was completed, the reaction mixture was diluted with water (100 mL) and extracted with dichloromethane (100 mL). The combined organic phases were washed with saturated sodium chloride solution (100 mL), dried over anhydrous sodium sulfate, filtered, concentrated under reduced pressure, and subjected to silica gel column chromatography (petroleum ether/ethyl acetate = 1/1 to ethyl acetate/methanol = 20/1) to obtain compound **6-5.** MS-ESI calculated for [M+H]⁺ 411, found 411.

### Step 5

Compound **6-5** (500 mg, 1.22 mmol) and compound **1-3** (206.07 mg, 1.46 mmol) were dissolved in dioxane (10 mL) and water (2 mL), then potassium carbonate (505.30 mg, 3.66 mmol) and [1,1'-bis(diphenylphosphino)ferrocene]dichloropalladium(II) (89.17 mg, 121.87 µmol) were added to the reaction mixture. Gas displacement was carried out and the reaction mixture was stirred at 130°C for 12 hours under a nitrogen atmosphere. After the reaction was completed, the reaction mixture was filtered, concentrated under reduced pressure, and subjected to high performance liquid chromatography (chromatographic column: Phenomene × luna C18 75 × 30 mm × 3 µm; mobile phase: mobile phase A: 0.225% formic acid aqueous solution by volume; mobile phase B: acetonitrile; B%: 22% to 52%) to obtain compound 6.

MS-ESI calculated for [M+H]⁺ 427, found 427. ¹H NMR (400 MHz, DMSO-*d*₆) δ ppm 8.77 (t, *J=* 1.6 Hz, 1 H), 8.66 (d, *J=* 2.8 Hz, 1 H), 8.27 (s, 1 H), 8.09 - 8.13 (m, 1 H), 8.07 (d, *J=* 2.8 Hz, 1 H), 7.93 (s, 1 H), 7.86 (d, *J =* 4.8 Hz, 1 H), 7.79 (d, *J =* 2.6 Hz, 1 H), 7.46 - 7.51 (m, 2 H), 7.38 - 7.43 (m, 2 H), 7.34 (d, *J=* 4.8 Hz, 1 H), 6.46 (d, *J=* 9.4 Hz, 1 H), 4.71 (d, *J=* 6.4 Hz, 2 H), 3.49 (s, 3 H).

### Example 7

### Synthetic route:

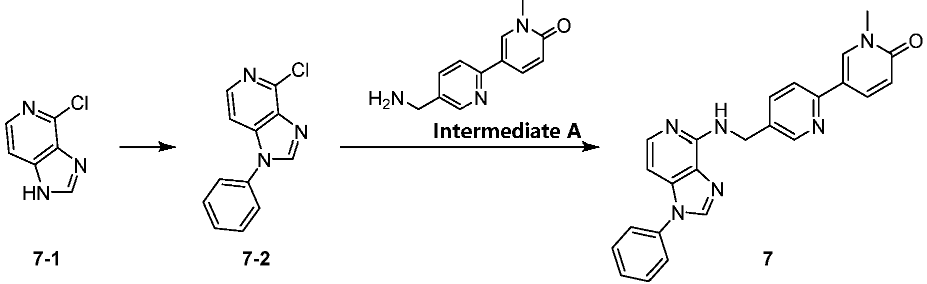

### Step 1

Compound **7-1** (1.5 g, 9.77 mmol) was dissolved in *N*,*N-*dimethylformamide (10 mL), and then phenylboronic acid (2.38 g, 19.54 mmol), copper acetate (3.55 g, 19.54 mmol), and pyridine (1.55 g, 19.54 mmol, 1.58 mL) were added thereto. The mixture was stirred in oxygen at 25°C for 12 hours. After the reaction was completed, aqueous ammonia solution (20 mL) and ethyl acetate (50 mL) were added to the reaction mixture, and stirred at 25°C for 50 minutes. The resulting mixture was added with water (30 mL) and extracted with ethyl acetate (50 mL × 3). The organic phase was washed with saturated brine (30 mL × 2), collected and dried over anhydrous sodium sulfate, filtered, and concentrated under reduced pressure to obtain compound **7-2.** MS-ESI calculated for [M+H]⁺ 230, found 230.

### Step 2

Compound **7-2** (200 mg, 870.83 µmol), **hydrochloride of compound intermediate A** (339.24 mg), and 1,8-diazabicyclo[5.4.0]undec-7-ene (530.29 mg , 3.48 µmol) were dissolved in N-methylpyrrolidone (3 mL). After the reaction was completed, the reaction mixture was added with water (20 mL) and extracted with ethyl acetate (20 mL × 3). The combined organic phases were dried over anhydrous sodium sulfate, filtered, and concentrated. The concentrate was subjected to thin-layer chromatography (developing solvent: dichloromethane/methanol = 10/1) to obtain a crude product, which was purified by high performance liquid chromatography (chromatographic column: Waters Xbridge C18 150 × 50 mm × 10 µm; mobile phase: mobile phase A: 10 mM ammonium bicarbonate aqueous solution; mobile phase B: acetonitrile; B%: 20% to 50%) to obtain compound **7.**

MS-ESI calculated for [M+H]⁺ 409, found 409. ¹H NMR (400 MHz, CD₃OD) δ 8.66 ppm (d, *J =* 1.8 Hz, 1H), 8.38 (d, *J =* 2.4 Hz, 1H), 8.35 (s, 1H), 8.19 (m, 1H), 7.92 (m, 1H), 7.84 (d, *J* = 6.2 Hz, 1H), 7.73 (d, *J =* 8.2 Hz, 1H), 7.69-7.60 (m, 4H), 7.59-7.53 (m, 1H), 6.92 (d, *J=* 6.2 Hz, 1H), 6.66 (d, *J=* 9.4 Hz, 1H), 4.88-4.87 (m, 2H), 3.68 (s, 3H).

### Example 8: Preparation of crystal form A of compound 2

100 mg of compound **2** was weighed and added to a 4.0 mL glass vial, and an appropriate amount of methanol was added to form a suspension. After adding a magneton, the suspension sample was placed on a magnetic heating stirrer (25°C) for testing. After stirring overnight at 25°C, the suspension sample was filtered, and the solid sample on the filter cake was placed in a vacuum drying oven (45°C) to dry overnight, resulting in the crystal form A of compound **2.** The XRPD, DSC, TGA, and DVS detection results of the crystal form A of compound **2** are shown in figures 1, 2, 3, and 4, respectively.

### Reference Example 9: Preparation of crystal form B of compound 2

100 mg of the crystal form A of compound **2** was weighed and placed in a microwave vial, and then placed in a muffle furnace and heated at 260°C for five minutes to obtain the crystal form B of the compound.

### Example 10: Study on solvent pre-stability of crystal form A of compound 2

100 mg of the crystal form A of compound 2 was weighed and added to a 4.0 mL glass vial, and an appropriate amount of solvent was added to form a suspension. (To ensure maximum suspension of the sample, the amount of compound and solvent was adjusted during the experiment based on the observed phenomena, and even the container used for the experiment was changed if necessary.) After adding a magneton, the suspension sample was placed on a magnetic heating stirrer (25°C) for testing. After stirring overnight at 25°C, the suspension sample was filtered, and the solid sample on the filter cake was placed in a vacuum drying oven (45°C) to dry overnight. The crystal form of the solid was obtained by X-ray powder diffraction (XRPD) detection. The test results are shown in Table 7.

**Table 7 Test results of solvent pre-stability of crystal form A of compound 2**

| Solvent | Crystal form | Solvent | Crystal form |
|---|---|---|---|
| Methanol | Crystal form A | Methyl *tert-butyl* ether | Crystal form A |
| Ethanol | Crystal form A | n-Heptane | Crystal form A |
| Ethyl acetate | Crystal form A | Isopropanol | Crystal form A |
| Acetone | Crystal form A | Acetone: water (2:1) | Crystal form A |
| Acetonitrile | Crystal form A | Ethanol: water (1:1) | Crystal form A |
| 2-Methyltetrahydrofuran | Crystal form A | Tetrahydrofuran | Crystal form A |
| Dioxane | Crystal form A | | |

**Conclusion:** The crystal form A of compound 2 has good stability in different solvents.

### Example 11: Study on hygroscopicity of crystal form A of compound 2

### Experimental materials:

SMS DVS intrinsic dynamic vapor sorption instrument

### Experimental methods:

10 to 30 mg of the crystal form A of compound **2** was taken and placed in the DVS sample tray for testing.

The classification of hygroscopicity evaluation is shown in Table 8.

**Table 8 Classification table for hygroscopicity evaluation**

| **Hygroscopicity classification** | **ΔW %** |
|---|---|
| Deliquesce | Absorb sufficient water to form a liquid |
| Extremely hygroscopic | ΔW% ≥ 15% |
| Hygroscopic | 15% > ΔW% ≥ 2% |
| Slightly hygroscopic | 2% > ΔW% ≥ 0.2% |
| No or almost no hygroscopicity | ΔW% < 0.2% |

| | |
|---|---|
| Note: ΔW% represents the increase in weight due to moisture absorption of the test sample at 25 ± 1°C and 80 ± 2% RH. | |

### Experimental results:

The DVS pattern of the crystal form A of compound 2 is shown in Figure 4. The test sample was tested according to the 30-95-0-95% RH/25°C procedure, with the sample pre-balanced at 30%RH. Compared to the initial humidity of 30%, when the humidity rose to 80%, the sample gained 0.07% in weight due to moisture absorption (cycle 1 adsorption); when the humidity continued to rise to 90%, the weight of the sample decreased by 0.15%; when the humidity continued to rise to 95%, the sample gained 0.01% in weight due to moisture absorption (cycle 1 adsorption). The XRPD test results showed that the diffraction peak positions before and after the DVS test were consistent, indicating that the crystal form did not change.

### Experimental Conclusion:

The crystal form A of compound **2** gained 0.07% in weight due to moisture absorption at 25°C and 80% RH, indicating no or almost no hygroscopicity.

### Example 12: Solid pre-stability test of crystal form A of compound 2

According to the Guiding Principles for Stability Testing of APIs and Formulations (Chinese Pharmacopoeia 2015 Edition, Part IV, General Rules 9001), the stability of the crystal form A of compound **2** was investigated under high temperature (60°C, open), high humidity (room temperature/relative humidity 92.5%, open), and light exposure (total illumination = 1.2 × 10⁶ Lux•hr/near ultraviolet = 200 w•hr/m², open) conditions.

10 mg of the crystal form A of compound **2** was weighed and placed at the bottom of a glass sample vial, spread into a thin layer. The sample placed under high temperature (60°C) and high humidity (relative humidity 92.5% RH) conditions was sealed with aluminum foil to seal the mouth of the vial, and small holes were made in the aluminum foil to ensure that the sample could fully contact the ambient air, and it was placed in the corresponding constant temperature and humidity box. The light sample (open, not covered with aluminum foil) and the light control sample (the entire sample vial was covered with aluminum foil) were placed in the light box. At each time point, two samples were weighed separately as official test samples. In addition, approximately 50 mg of the crystal form A of compound **2** was weighed for XRPD testing. The sample vial was wrapped with aluminum foil and small holes were made, and it was also placed in the corresponding constant temperature and humidity box. The sample was taken for testing (XRPD) on days 5 and 10, and the test results were compared with the initial test results on day 0. The results of the solid pre-stability test of crystal form A of compound **2** are shown in Table 9.

**Table 9 Test results of solid pre-stability of crystal form A of compound 2**

| Stability study conditions | **Crystal form** |
|---|---|
| 0 days | Crystal form A |
| High temperature (60°C, open), 5 days | Crystal form A |
| High temperature (60°C, open), 10 days | Crystal form A |
| High humidity (relative humidity 92.5%, open), 5 days. | Crystal form A |
| High humidity (relative humidity 92.5%, open), 10 days. | Crystal form A |
| Light (total illumination = 1.2 × 10⁶ Lux•hr/near ultraviolet = 200 w•hr/m², open), 5 days | Crystal form A |
| Light (total illumination = 1.2 × 10⁶ Lux•hr/near ultraviolet = 200 w•hr/m², open), 10 days | Crystal form A |

**Conclusion:** The crystal form A of compound **2** has good stability under the conditions of high temperature, high humidity, and strong light.

### Biological evaluation:

### Experimental example 1: Super-Top-Flash (STF) reporter gene inhibitory activity test

### Experimental materials:

HEK293 STF and L wnt3A cells were provided by Wuhan Heyan Biomedical Technology Co., Ltd., and Bright Glo was purchased from Promega.

### Experimental methods:

HEK293 STF and L wnt3A cells were seeded in a white 96-well plate at a ratio of 1:1, with 80 µL of cell suspension per well, each containing 20,000 HEK293 STF cells and 20,000 L wnt3A cells. The cell plate was placed in a carbon dioxide incubator for overnight culture.

The test compounds were 5-fold diluted with a multi-channel pipette to the 8th concentration, i.e., diluted from 400 µM to 5.12 nM, and duplicate experiment was set. 78 µL of culture medium was added to an intermediate plate, and then 2 µL of gradient diluted compound per well was transferred to the intermediate plate according to the corresponding position. After mixing well, the compound was transferred to the cell plate at 20 µL per well. The cell plate was placed in a carbon dioxide incubator and cultured for 24 hours.

100 µL of Promega Bright-Glo reagent was added to each well of the cell plate, and it was incubated at room temperature for 10 minutes to stabilize the luminescent signal. A multimode microplate reader was used for reading.

Using the raw data from the multimode microplate reader, the IC₅₀ value could be obtained through curve fitting. Table 10 provides the data on the inhibitory activity of the compounds of the present disclosure on the Wnt signaling pathway. The results shows that the compounds of the present disclosure have good inhibitory activity on the Porcupine of the Wnt signaling pathway.

**Table 10 Test results of inhibitory activity of compounds of the present disclosure on the WNT pathway STF reporter gene**

| **Compound** | **IC₅₀ (nM)** |
|---|---|
| Compound 1 | 8.41 |
| Compound 2 | 0.23 |

### Experimental example 2: Capan-2 cell anti-proliferative activity test

### Experimental materials:

Capan-2 cells were provided by Wuhan Heyan Biomedical Technology Co., Ltd., and CellTiter-Glo was purchased from Promega.

### Experimental methods:

Capan-2 cells were seeded in a white 96-well plate, with 80 µL of cell suspension per well, each containing 5,000 Capan-2 cells. The cell plate was placed in a carbon dioxide incubator for overnight culture.

The test compounds were 3-fold diluted with a multi-channel pipette to the 9th concentration, i.e., diluted from 200 µM to 30 nM, and duplicate experiment was set. 78 µL of culture medium was added to an intermediate plate, and then 2 µL of gradient diluted compound per well was transferred to the intermediate plate according to the corresponding position. After mixing well, the compound was transferred to the cell plate at 20 µL per well. The cell plate was placed in a carbon dioxide incubator and cultured for 5 days. Another cell plate was prepared, and the signal value was read on the day of drug administration to serve as the maximum value (Max value in the equation below) for data analysis. 25 µL of cell viability chemiluminescence detection reagent was added to each well of this cell plate, and it was incubated at room temperature for 10 minutes to stabilize the luminescent signal. A multimode microplate reader was used for reading.

After the cell plate was cultured for 5 days, 25 µL of Promega CellTiter-Glo reagent was added to each well of the cell plate, and it was incubated at room temperature for 10 minutes to stabilize the luminescent signal. A multimode microplate reader was used for reading.

The IC₅₀ value could be obtained through curve fitting, and the results of the inhibitory activity of the compounds of the present disclosure on the proliferation of Capan-2 cells are shown in Table 11.

**Table 11 Test results of the anti-proliferative activity of the compounds of the present disclosure on Capan-2 cells**

| **Compound** | **IC₅₀ (nM)** | **Compound** | **IC₅₀ (nM)** |
|---|---|---|---|
| Compound 1 | 6.09 | Compound 5 | 11.7 |
| Compound 2 | 6.07 | Compound 6 | 22.24 |
| Compound 3 | 27.99 | Compound 7 | 5.94 |
| Compound 4 | 24.6 | | |

Experimental conclusion: The compounds of the present disclosure have good inhibitory activity on the proliferation of Capan-2 cells.

### Experimental example 3: In vivo drug efficacy experiment in HuPrime^{®} gastric cancer GA3055 subcutaneous xenograft model in female BALB/C nude mice

### Experimental materials:

GA3055 tumor-bearing mice were provided by Crown Bioscience (Taicang) Co., Ltd.

### Experimental methods:

Tumor tissue was collected from tumor-bearing mice of the HuPrime^{®} gastric cancer GA3055 xenograft model, and cut into tumor pieces with a diameter of 2 to 3 mm for subcutaneous inoculation in the right anterior scapular region of BALB/c nude mice. When the average tumor volume in the tumor-bearing mice reached approximately 150 mm³, the mice were randomly grouped. The coefficient of variation (CV) of the tumor volume within each group was calculated using the formula CV = SD / MTV × 100%, and should be less than 30%. Administration started on the day of grouping (the tumor volume was measured on the day of grouping). The day of grouping was defined as day 0.

Before the start of administration, all animals were weighed and the tumor volume was measured using a caliper. Given that the volume of the tumor could affect the effectiveness of the treatment, a random grouping design method was used. The mice were grouped based on the volume of their tumors to ensure similar tumor volumes across different groups. Grouping was done using StudyDirectorTM (version 3.1.399.19, supplier: Studylog System, Inc., S. San Francisco, CA, USA).

The "Matched distribution" random grouping method was chosen for grouping, which could reflect the smallest intergroup differences at the tumor volume level. This algorithm matched the individual measurements of all selected animals with the average value of all selected animals. Firstly, those paired animals whose average values were close to the average value of all selected animals were chosen, and then they were allocated to groups so that the average value of the group matched (or was as close as possible to) the average value of all selected animals. The final average value of the measurements of each group was as close as possible to the final average value of the other groups.

After the inoculation of tumor cells, routine monitoring included the growth of the tumor and the impact of the treatment on the normal behavior of the animals. Specific content included the activity of the experimental animals, food and water intake, weight gain or loss, and any abnormalities in their eyes, fur, and other conditions. Any clinical symptoms observed during the experiment were recorded in the original data. After the start of administration, the weight of the mice and the size of the tumor were measured twice a week. The formula for calculating tumor volume was: Tumor volume (mm³) = 1/2 × (a × b²) (where a represents the long diameter, and b represents the short diameter). During the experiment, data was collected using the StudyDirectorTM software (version 3.1.399.19, supplier: Studylog System, Inc.), including measurements of the long and short diameters of the tumor and the weight of the animals. The original data was directly imported into the software after being measured by a balance and caliper, and any changes in the data were recorded in this software. All processes, including administration, tumor measurement, and weight measurement, were carried out in a biosafety cabinet or ultra-clean workbench.

All experimental results were expressed as the mean tumor volume ± SEM (standard error of the mean). The optimal drug treatment point (usually after the last administration) was chosen for statistical analysis between different groups. An independent sample T-test was used to compare whether there was a significant difference in the relative tumor volume and tumor weight of the treatment group compared to the control group. All data were analyzed using SPSS 18.0. A p-value of < 0.05 was considered statistically significant.

The experimental results showed that the compound of the present disclosure, at a dose of 5 mg/Kg BID (twice daily), administered for 37 days, had a significant tumor inhibitory effect in the *in vivo* drug efficacy experiment on the HuPrime^{®} gastric cancer GA3055 subcutaneous xenograft model in female BALB/C nude mice, as shown in Table 12.

**Table 12 Anti-tumor efficacy of the compound of the present disclosure in the HuPrime^{®} gastric cancer GA3055 subcutaneous xenograft model in female BALB/C nude mice**

| **Compound** | **Dose** | **TGI%** | **T/C%** | **P value** |
|---|---|---|---|---|
| Compound 2 | 5 mg/Kg, BID | 94.6 | 12.97 | < 0.001 compared with vehicle group |
| Compound 2 | 10 mg/Kg, BID | 96.8 | 10.89 | < 0.001 compared with |
| | | | | vehicle group |

Experimental conclusion: The compound of the present disclosure has a good tumor inhibitory effect in the HuPrime^{®} gastric cancer GA3055 subcutaneous xenograft model in female BALB/C nude mice.

### Experimental example 4: Pharmacokinetic evaluation

### Experimental materials:

CD-1 mice (male, 7 to 9 weeks old, Shanghai SLAC)

### Experimental operation:

The pharmacokinetic characteristics of the compound in rodents were tested using a standard protocol after intravenous injection (IV) and oral (PO) administration. In the experiment, mice were given a single intravenous injection and oral administration. The solvent for the intravenous injection was water. The solvent for oral administration was 0.5% hydroxypropyl methylcellulose. This project involved four male CD-1 mice. Two of the mice were administered a dose of 0.5 mg/kg via intravenous injection. Plasma samples were collected at 0 hours (before administration) and at 0.083, 0.25, 0.5, 1, 2, 4, 8, and 24 hours after administration. The other two mice were administered a dose of 2.5 mg/kg via oral gavage. Plasma samples were collected at 0 h (before administration) and at 0.25, 0.5, 1, 2, 4, 8, and 24 hours after administration. Whole blood samples were collected within 24 hours, centrifuged at 3000 g for 15 minutes, and the supernatant was separated to obtain plasma samples. Four times the volume of acetonitrile solution containing an internal standard was added to precipitate the protein. After centrifugation, the supernatant was collected, an equal volume of water was added, and it was centrifuged again to collect the supernatant for sampling. The plasma concentration was quantitatively analyzed by LC-MS/MS analysis method, and the pharmacokinetic parameters were calculated, such as peak concentration (Cₘₐₓ), clearance rate (CL), half-life (T_{1/2}), volume of distribution at steady state (Vdss), area under drug-time curve (AUC₀₋ₗₐₛₜ), bioavailability (F).

The experimental results are shown in Table 13.

**Table 13 Pharmacokinetic evaluation results of the compounds of the present disclosure in mice**

| **Compound** | **F (%)** | **Cₘₐₓ (nM)** | **T_{1/2} (IV) (hr)** | **Vdss (L/kg)** | **CL (mL/min/k g)** | **AUC₀₋ₗₐₛₜ (PO) (nM·hr)** |
|---|---|---|---|---|---|---|
| Compound 2 | 29.4 | 1755 | 5.99 | 0.568 | 5.37 | 20518 |

Experimental conclusion: The compounds of the present disclosure have good pharmacokinetic properties.

### Experimental example 5: Toxicological evaluation experiment of rats administered for 21 consecutive days

### Experimental materials:

SD rats (male and female, 7 to 8 weeks old, Zhejiang Vital River Laboratory Animal Technology Co., Ltd.).

### Experimental operation:

In this experiment, SD rats were administered orally once a day with a test substance, a candidate drug for treating cancer, for 21 days to detect its potential toxicity. At the same time, the toxicokinetic (TK) characteristics of the compound in the animals were examined.

126 rats (63 per gender) were divided into 6 groups. In the main test administration groups, each group had 5 males and 5 females. In the TK test control group, there were 3 males and 3 females. In the administration groups, each group had 6 males and 6 females. The compound was administered once a day at doses of 1, 3, 10, 20 milligrams per kilogram per day (mg/kg/day). The control group was only given the solvent. The volume of administration was 10 mL/kg. At the start of administration, the animals were 7 to 8 weeks old, with male weights ranging from 251.11 to 308.11 grams, and female weights from 181.53 to 219.03 grams.

The evaluation items include mortality (moribund/death), clinical symptoms, body weight, food consumption, clinical pathology (hematology, serum biochemistry, coagulation), gross (necropsy) evaluation, organ weights, histopathological evaluation, and toxicokinetics. The toxicokinetic parameters include peak concentration (Cₘₐₓ), time to maximum drug concentration (Tₘₐₓ), and area under the drug-time curve (AUC₀₋ₗₐₛₜ).

The experimental results are shown in Table 14.

**Table 14 Toxicokinetic evaluation results of the compound of the present disclosure administered to rats for 21 consecutive days**

| **Dosage of compound 2** | | **1 mg/kg/day** | | **3 mg/kg/day** | | **10 mg/kg/day** | | **20 mg/kg/day** | |
|---|---|---|---|---|---|---|---|---|---|
| **Sampling time** | | Day 1 | Day 21 | Day 1 | Day 21 | Day 1 | Day 21 | Day 1 | Day 21 |
| **Male** | Tmax (hr) | 8 | 8 | 8 | 8 | 4 | 8 | 8 | 8 |
| | Cmax (nM) | 970 | 1623 | 1881 | 3502 | 2963 | 6870 | 4243 | 7341 |
| | AUC₀₋₂₄ₕ **(nM·hr)** | 12505 | 22428 | 23124 | 50065 | 47371 | 109784 | 76557 | 123030 |
| **Female** | Tmax (hr) | 4 | 8 | 8 | 8 | 4 | 8 | 8 | 8 |
| | Cmax (nM) | 1024 | 1578 | 2268 | 4176 | 3929 | 8801 | 6174 | 10327 |
| | AUC₀₋₂₄ₕ **(nM·hr)** | 14503 | 25594 | 33227 | 63760 | 77679 | 143685 | 100804 | 178708 |

Experimental conclusion: During the administration of the compound of the present disclosure, there was no significant decrease in animal weight, and the tolerance was good. Compared with the vehicle control group, the main observations were a slight increase in fibrinogen and total bilirubin, a decrease in trabecular bone/cortical bone, thickening of the growth plate, and no other obvious abnormalities. In the toxicological evaluation of the compound of the present disclosure administered to rats for 21 consecutive days, the maximum tolerated dose and the maximum tolerated exposure were high, indicating good safety of the compound of the present disclosure.

## Claims

1. A compound of formula (X) or a pharmaceutically acceptable salt thereof, 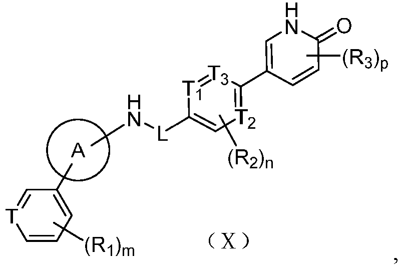 wherein
T is selected from CR or N;
T₁ is selected from CH or N;
T₂ is selected from CH or N;
T₃ is selected from CH or N;
L is selected from a single bond and -C(R₄R₅)-;
ring A is 9- to 10-membered heteroaryl, wherein the 9- to 10-membered heteroaryl is optionally substituted by 1, 2, or 3 Rₐ;
each R₁ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{b};
each R₂ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{c};
each R₃ is independently selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{d};
R₄ and R₅ are each independently selected from H and C₁₋₃ alkyl, wherein the C₁₋₃ alkyl is optionally substituted by 1, 2, or 3 Rₑ;
R is selected from H, F, Cl, Br, I, -CN, -OH, -NH₂, C₁₋₃ alkyl, C₁₋₃ alkoxy, and C₁₋₃ alkylamino, wherein the C₁₋₃ alkyl, C₁₋₃ alkoxy, and C₁₋₃ alkylamino are each independently and optionally substituted by 1, 2, or 3 R_{f};
each Rₐ is independently selected from F, Cl, Br, I, -OH, -NH₂, and -CN;
each R_{b} is independently selected from F, Cl, Br, I, -OH, -NH₂, and -CN;
each R_{c} is independently selected from F, Cl, Br, I, -OH, -NH₂, and -CN;
each R_{d} is independently selected from F, Cl, Br, I, -OH, -NH₂, and -CN;
each Rₑ is independently selected from F, Cl, Br, I, -OH, -NH₂, and -CN;
each R_{f} is independently selected from F, Cl, Br, I, -OH, -NH₂, and -CN;
m is selected from 0, 1, 2, and 3;
n is selected from 0, 1, 2, and 3;
p is selected from 0, 1, 2, and 3;
"hetero" in the 9- to 10-membered heteroaryl represents 1, 2, 3, or 4 heteroatoms or heteroatom groups independently selected from O, S, and N.

2. The compound or the pharmaceutically acceptable salt thereof according to claim 1, wherein the compound has a structure of formula (X-1): 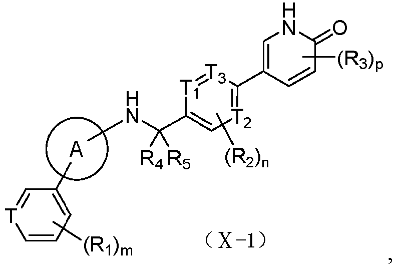 wherein ring A, R₁, R₂, R₃, R₄, R₅, T, T₁, T₂, T₃, m, n, and p are as defined in claim 1.

3. The compound or the pharmaceutically acceptable salt thereof according to claim 1 or 2, wherein each R₁ is independently selected from H, F, Cl, Br, I, -CN, -OH, -NH₂, -CH₃, and -OCH₃, wherein the -CH₃ and -OCH₃ are each independently and optionally substituted by 1, 2, or 3 R_{b};
or, each R₂ is independently selected from H;
or, each R₃ is independently selected from H, F, Cl, Br, I, -CN, -OH, -NH₂, and -CH₃, wherein the -CH₃ is optionally substituted by 1, 2, or 3 R_{d};
or, R₄ and R₅ are each independently selected from H and -CH₃, wherein the -CH₃ is optionally substituted by 1, 2, or 3 Rₑ;
or, R is selected from H, F, Cl, Br, I, -CN, -OH, -NH₂, and -CH₃, wherein the -CH₃ is optionally substituted by 1, 2, or 3 R_{f};
or, ring A is selected from 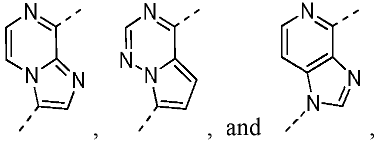 wherein 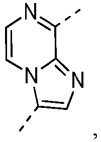 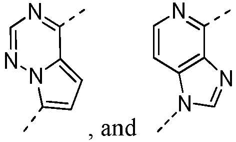 are each independently and optionally substituted by 1, 2, or 3 Rₐ.

4. The compound or the pharmaceutically acceptable salt thereof according to claim 3, wherein each R₁ is independently selected from H, F, -CH₃, and -OCH₃;
or, each R₃ is independently selected from -CH₃;
or, R₄ and R₅ are each independently selected from H;
or, R is selected from H and F;
or, ring A is selected from

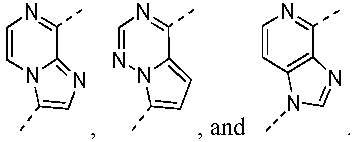

5. The compound or the pharmaceutically acceptable salt thereof according to claim 1 or 2, wherein the compound has a structure of formula (X-2) or (X-3): 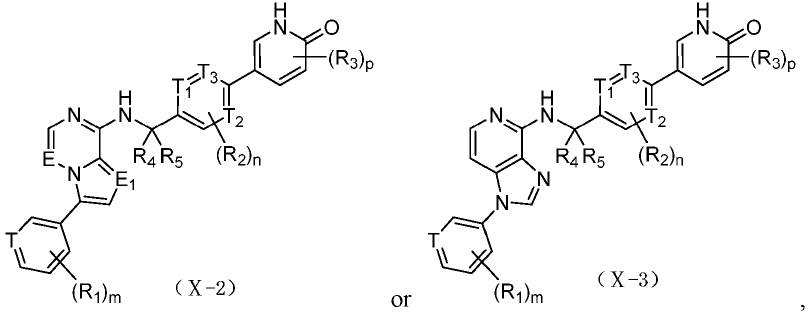 wherein
E is selected from CH and N;
E₁ is selected from CH and N;
R₁, R₂, R₃, R₄, R₅, T, T₁, T₂, T₃, m, n, and p are as defined in claim 1 or 2.

6. The compound or the pharmaceutically acceptable salt thereof according to claim 5, wherein the compound has a structure of formula (X-4), (X-5), or (X-6): 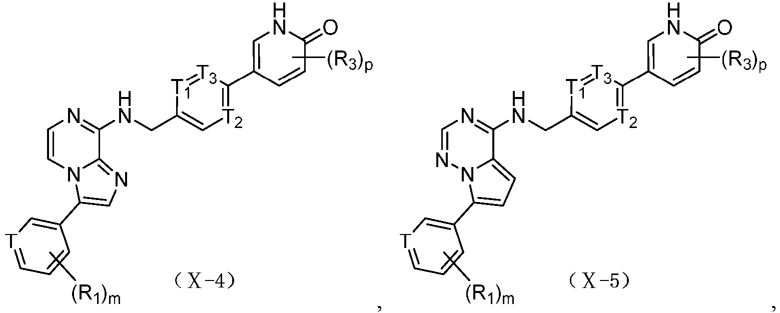 or 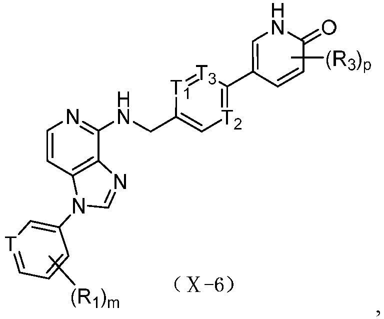 wherein R₁, R₃, T, T₁, T₂, T₃, m, and p are as defined in claim 5.

7. The compound or the pharmaceutically acceptable salt thereof according to claim 6, wherein the compound has a structure of formulas (X-7), (X-8), and (X-9): 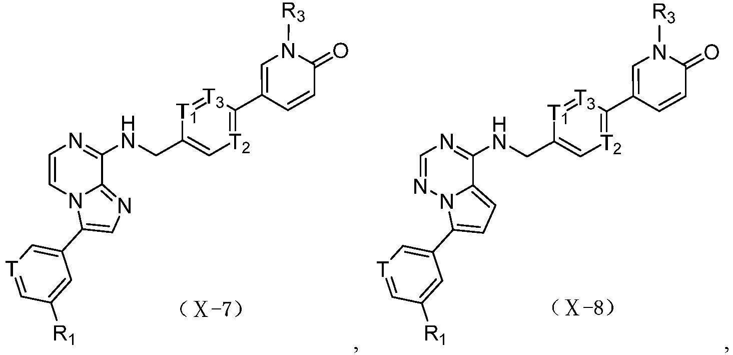 or 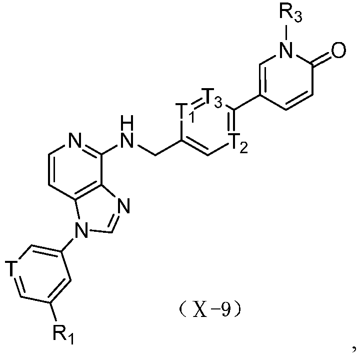 wherein R₁, R₃, T, T₁, T₂, and T₃ are as defined in claim 6.

8. The compound or the pharmaceutically acceptable salt thereof according to claim 1, wherein the compound is selected from the following formula,

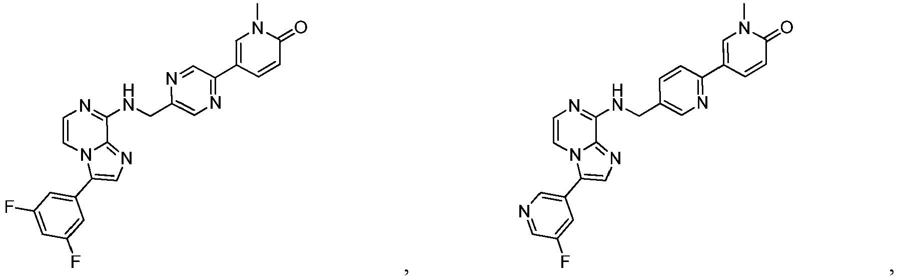

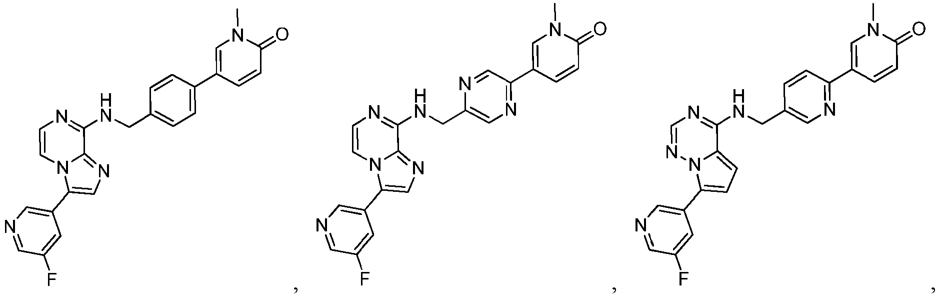

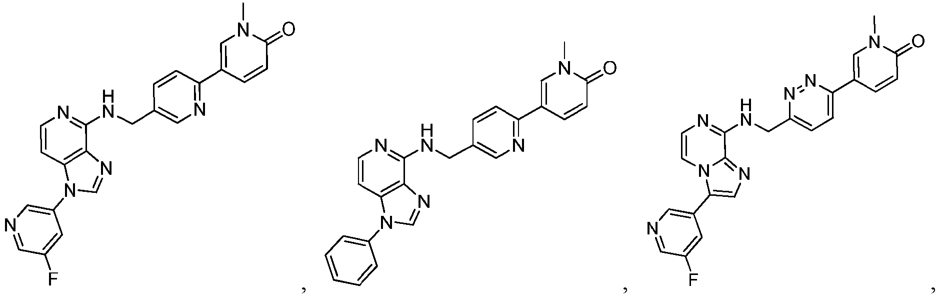

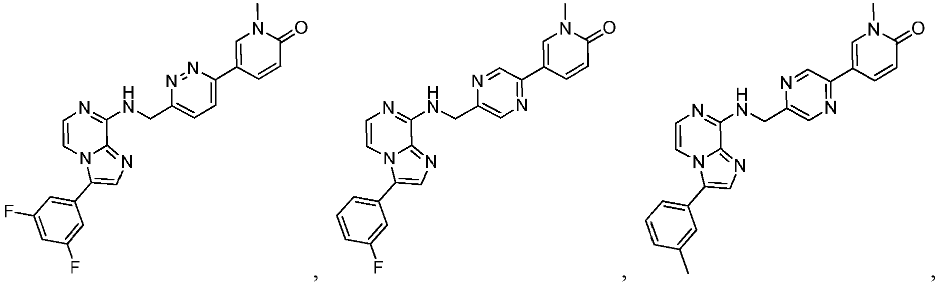

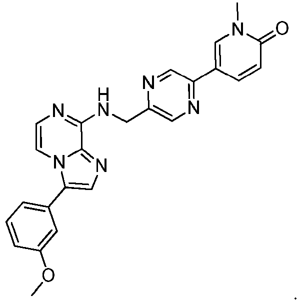

9. A crystal form A of compound 2 according to claim 8, wherein the crystal form A has an X-ray powder diffraction pattern comprising characteristic diffraction peaks at the following 2θ angles: 7.45 ± 0.20°, 13.53 ± 0.20°, 13.94 ± 0.20°, and 15.93 ± 0.20°;

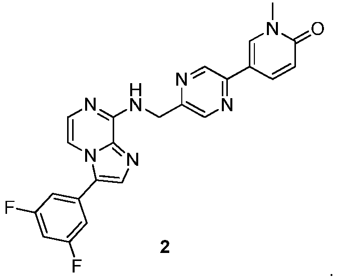

10. The crystal form A according to claim 9, wherein the X-ray powder diffraction pattern of the crystal form A comprises characteristic diffraction peaks at the following 2θ angles: 7.45 ± 0.20°, 10.56 ± 0.20°, 13.53 ± 0.20°, 13.94 ± 0.20°, 14.86 ± 0.20°, 15.93 ± 0.20°, and 17.96 ± 0.20°;
preferably, the X-ray powder diffraction pattern of the crystal form A comprises characteristic diffraction peaks at the following 2θ angles: 4.96 ± 0.20°, 7.45 ± 0.20°, 10.56 ± 0.20°, 13.53 ± 0.20°, 13.94 ± 0.20°, 14.86 ± 0.20°, 15.93 ± 0.20°, 17.96 ± 0.20°, 19.87 ± 0.20°, 20.90 ± 0.20°, 25.38 ± 0.20°, and 28.02 ± 0.20°;
more preferably, the X-ray powder diffraction pattern of the crystal form A comprises characteristic diffraction peaks at the following 2θ angles: 4.96 ± 0.20°, 7.45 ± 0.20°, 10.56 ± 0.20°, 13.53 ± 0.20°, 13.94 ± 0.20°, 14.86 ± 0.20°, 15.93 ± 0.20°, 17.96 ± 0.20°, 19.06 ± 0.20°, 19.87 ± 0.20°, 20.90 ± 0.20°, 24.11 ± 0.20°, 24.97 ± 0.20°, 25.38 ± 0.20°, and 28.02 ± 0.20°;
further preferably, the X-ray powder diffraction pattern of the crystal form A comprises characteristic diffraction peaks at the following 2θ angles: 4.96°, 7.45°, 10.56°, 13.53°, 13.94°, 14.86°, 15.93°, 17.56°, 17.96°, 19.06°, 19.87°, 20.90°, 21.21°, 21.90°, 22.63°, 24.11°, 24.97°, 25.38°, 25.96°, 27.14°, 28.02°, 28.73°, 29.94°, 30.89°, 32.50°, 34.01°, 35.05°, 35.95°, 37.54°, and 39.08°.

11. A crystal form A according to claim 9 or 10, having XRPD pattern analysis data as shown in the following table,
| No. | 2θ angle [°] | Peak height [cts] | Left full width at half maximum [°2θ] | Interplanar spacing [Å] | Relative intensity [%] |
|---|---|---|---|---|---|
| 1 | 4.96 | 798.52 | 0.1279 | 17.80 | 5.32 |
| 2 | 7.45 | 1349.94 | 0.1535 | 11.87 | 9.00 |
| 3 | 10.56 | 873.60 | 0.1791 | 8.37 | 5.83 |
| 4 | 13.53 | 4986.93 | 0.1791 | 6.55 | 33.25 |
| 5 | 13.94 | 14996.96 | 0.1791 | 6.35 | 100.00 |
| 6 | 14.86 | 1270.50 | 0.1791 | 5.96 | 8.47 |
| 7 | 15.93 | 3547.78 | 0.1791 | 5.56 | 23.66 |
| 8 | 17.56 | 432.50 | 0.1791 | 5.05 | 2.88 |
| 9 | 17.96 | 1770.00 | 0.1791 | 4.94 | 11.80 |
| 10 | 19.06 | 435.51 | 0.1535 | 4.66 | 2.90 |
| 11 | 19.87 | 899.45 | 0.2047 | 4.47 | 6.00 |
| 12 | 20.90 | 460.45 | 0.1535 | 4.25 | 3.07 |
| 13 | 21.21 | 423.35 | 0.1535 | 4.19 | 2.82 |
| 14 | 21.90 | 104.94 | 0.2558 | 4.06 | 0.70 |
| 15 | 22.63 | 291.20 | 0.1279 | 3.93 | 1.94 |
| 16 | 24.11 | 596.83 | 0.1791 | 3.69 | 3.98 |
| 17 | 24.97 | 871.02 | 0.2047 | 3.57 | 5.81 |
| 18 | 25.38 | 979.35 | 0.2047 | 3.51 | 6.53 |
| 19 | 25.96 | 879.75 | 0.1791 | 3.43 | 5.87 |
| 20 | 27.14 | 280.14 | 0.2047 | 3.29 | 1.87 |
| 21 | 28.02 | 877.39 | 0.2047 | 3.18 | 5.85 |
| 22 | 28.73 | 379.96 | 0.1535 | 3.11 | 2.53 |
| 23 | 29.94 | 275.89 | 0.4093 | 2.98 | 1.84 |
| 24 | 30.89 | 224.53 | 0.2047 | 2.89 | 1.50 |
| 25 | 32.50 | 309.77 | 0.2047 | 2.75 | 2.07 |
| 26 | 34.01 | 178.62 | 0.2558 | 2.64 | 1.19 |
| 27 | 35.05 | 248.70 | 0.1535 | 2.56 | 1.66 |
| 28 | 35.95 | 67.09 | 0.3070 | 2.50 | 0.45 |
| 29 | 37.54 | 224.08 | 0.2303 | 2.40 | 1.49 |
| 30 | 39.08 | 170.32 | 0.2047 | 2.30 | 1.14 |

12. The crystal form A according to any one of claims 9 to 11, wherein the crystal form A has a differential scanning calorimetry (DSC) curve comprising onsets of endothermic peaks at 259.7°C ± 5°C and 274.7°C ± 5°C.

13. The crystal form A according to any one of claims 9 to 11, wherein the crystal form A has a thermogravimetric analysis (TGA) curve with a weight loss of 0.80% at 240°C ± 3°C.

14. The compound or the pharmaceutically acceptable salt thereof according to any one of claims 1 to 8, or the crystal form A according to any one of claims 9 to 13 for use in the inhibition of porcupine.

15. The compound or the pharmaceutically acceptable salt thereof according to any one of claims 1 to 8, or the crystal form A according to any one of claims 9 to 13 for use in the treatment of pancreatic cancer, colorectal cancer, and/or gastric cancer.

## Patentansprüche

1. Verbindung der Formel (X) oder pharmazeutisch annehmbares Salz davon, 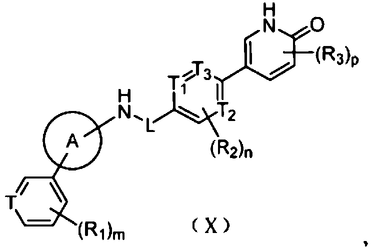 worin
T aus CR und N ausgewählt ist;
T₁ aus CH und N ausgewählt ist;
T₂ aus CH und N ausgewählt ist;
T₃ aus CH und N ausgewählt ist;
L aus einer Einfachbindung und -C(R₄R₅)- ausgewählt ist;
Ring A ein 9- bis 10-gliedriges Heteroaryl ist, wobei das 9- bis 10-gliedrige Heteroaryl gegebenenfalls mit 1, 2 oder 3 Rₐ substituiert ist;
die R₁ jeweils unabhängig aus H, F, Cl, Br, I, -CN, -OH, -NH₂, C₁₋₃-Alkyl, C₁₋₃-Alkoxy und C₁₋₃-Alkylamino ausgewählt sind, wobei das C₁₋₃-Alkyl, C₁₋₃-Alkoxy und C₁₋₃-Alkylamino jeweils unabhängig und gegebenenfalls mit 1, 2 oder 3 R_{b} substituiert sind;
die R₂ jeweils unabhängig aus H, F, Cl, Br, I, -CN, -OH, -NH₂, C₁₋₃-Alkyl, C₁₋₃-Alkoxy und C₁₋₃-Alkylamino ausgewählt sind, wobei das C₁₋₃-Alkyl, C₁₋₃-Alkoxy und C₁₋₃-Alkylamino jeweils unabhängig und gegebenenfalls mit 1, 2 oder 3 R_{c} substituiert sind;
die R₃ jeweils unabhängig aus H, F, Cl, Br, I, -CN, -OH, -NH₂, C₁₋₃-Alkyl, C₁₋₃-Alkoxy und C₁₋₃-Alkylamino ausgewählt sind, wobei das C₁₋₃-Alkyl, C₁₋₃-Alkoxy und C₁₋₃-Alkylamino jeweils unabhängig und gegebenenfalls mit 1, 2 oder 3 R_{d} substituiert sind;
R₄ und R₅ jeweils unabhängig aus H und C₁₋₃-Alkyl ausgewählt sind, wobei das C₁₋₃-Alkyl gegebenenfalls mit 1,2 oder 3 R_{c} substituiert ist;
R aus H, F, Cl, Br, I, -CN, -OH, -NH₂, C₁₋₃-Alkyl, C₁₋₃-Alkoxy und C₁₋₃-Alkylamino ausgewählt ist, wobei das C₁₋₃-Alkyl, C₁₋₃-Alkoxy und C₁₋₃-Alkylamino jeweils unabhängig und gegebenenfalls mit 1, 2 oder 3 R_{f} substituiert sind;
die Rₐ jeweils unabhängig aus F, Cl, Br, I, -OH, -NH₂ und -CN ausgewählt sind;
die R_{b} jeweils unabhängig aus F, CI, Br, I, -OH, -NH₂ und -CN ausgewählt sind;
die R_{c} jeweils unabhängig aus F, Cl, Br, I, -OH, -NH₂ und -CN ausgewählt sind;
die R_{d} jeweils unabhängig aus F, Cl, Br, I, -OH, -NH₂ und -CN ausgewählt sind;
die Rₑ jeweils unabhängig aus F, Cl, Br, I, -OH, -NH₂ und -CN ausgewählt sind;
die R_{f} jeweils unabhängig aus F, Cl, Br, I, -OH, -NH₂ und -CN ausgewählt sind;
m aus 0, 1, 2 und 3 ausgewählt ist;
n aus 0, 1, 2 und 3 ausgewählt ist;
p aus 0, 1, 2 und 3 ausgewählt ist;
"Hetero" in dem 9- bis 10-gliedrigen Heteroaryl für 1, 2, 3 oder 4 Heteroatome oder Heteroatomgruppen steht, die jeweils unabhängig aus O, S und N ausgewählt sind.

2. Verbindung oder pharmazeutisch annehmbares Salz davon nach Anspruch 1, wobei die Verbindung eine Struktur der Formel (X-1) aufweist: 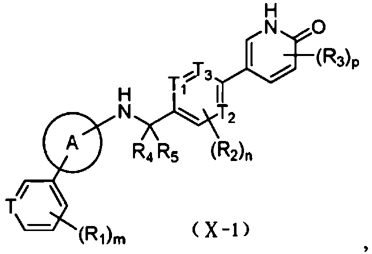 worin Ring A, R₁, R₂, R₃, R₄, R₅, T, T₁, T₂, T₃, m, n und p wie in Anspruch 1 definiert sind.

3. Verbindung oder pharmazeutisch annehmbares Salz davon nach Anspruch 1 oder 2, wobei die R₁ jeweils unabhängig aus H, F, Cl, Br, I, -CN, -OH, -NH₂, -CH₃ und -OCH₃ ausgewählt sind, wobei das -CH₃ und -OCH₃ jeweils unabhängig und gegebenenfalls mit 1, 2 oder 3 R_{b} substituiert sind;
oder die R₂ jeweils unabhängig aus H ausgewählt sind;
oder die R₃ jeweils unabhängig aus H, F, Cl, Br, I, -CN, -OH, -NH₂ und -CH₃ ausgewählt sind, wobei das -CH₃ gegebenenfalls mit 1, 2 oder 3 R_{d} substituiert ist;
oder R₄ und R₅ jeweils unabhängig aus H und -CH₃ ausgewählt sind, wobei das -CH₃ gegebenenfalls mit 1, 2 oder 3 Rₑ substituiert ist;
oder R aus H, F, Cl, Br, I, -CN, -OH, -NH₂ und -CH₃ ausgewählt ist, wobei das -CH₃ gegebenenfalls mit 1, 2 oder 3 R_{f} substituiert ist,
oder Ring A aus 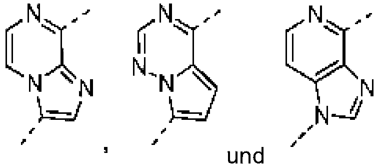 ausgewählt ist, wobei 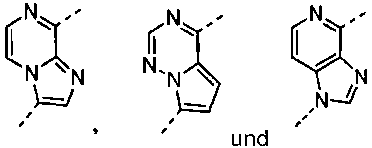 jeweils unabhängig und gegebenenfalls mit 1, 2 oder 3 Rₐ substituiert sind.

4. Verbindung oder pharmazeutisch annehmbares Salz davon nach Anspruch 3, wobei die R₁ jeweils unabhängig aus H, F, -CH₃ und -OCH₃ ausgewählt sind;
oder die R₃ jeweils unabhängig aus -CH₃ ausgewählt sind;
oder R₄ und R₅ jeweils unabhängig aus H ausgewählt sind;
oder R aus H und F ausgewählt ist;
oder Ring A aus 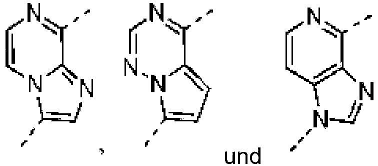 ausgewählt ist.

5. Verbindung oder pharmazeutisch annehmbares Salz davon nach Anspruch 1 oder 2, wobei die Verbindung eine Struktur der Formel (X-2) oder (X-3) aufweist: worin 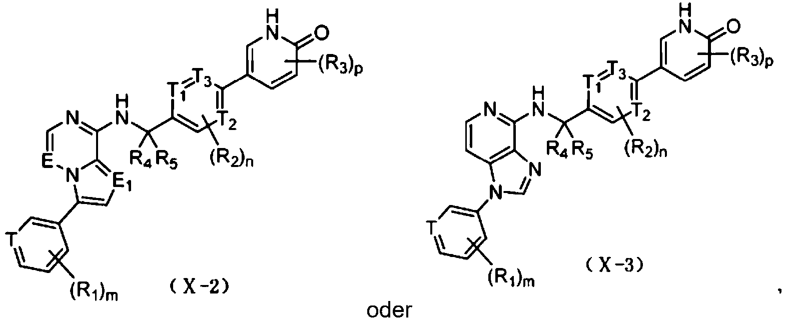
E aus CH und N ausgewählt ist;
E₁ aus CH und N ausgewählt ist;
R₁, R₂, R₃, R₄, R₅, T, T₁, T₂, T₃, m, n und p wie in Anspruch 1 oder 2 definiert sind.

6. Verbindung oder pharmazeutisch annehmbares Salz davon nach Anspruch 5, wobei die Verbindung eine Struktur der Formel (X-4), (X-5) oder (X-6) aufweist: 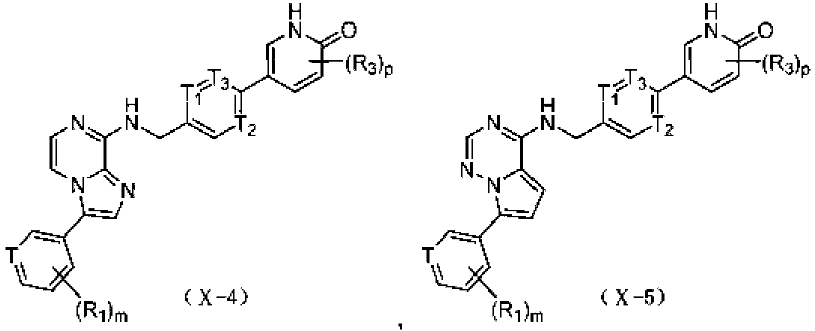 oder 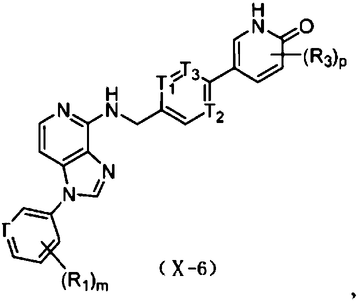 worin R₁, R₃, T, T₁, T₂, T₃, m und p wie in Anspruch 5 definiert sind.

7. Verbindung oder pharmazeutisch annehmbares Salz davon nach Anspruch 6, wobei die Verbindung eine Struktur der Formeln (X-7), (X-8) und (X-9) aufweist: 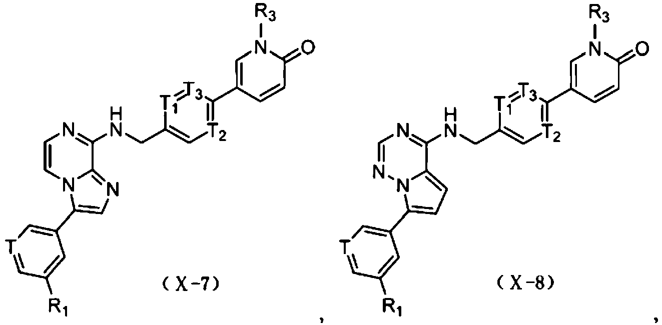 oder 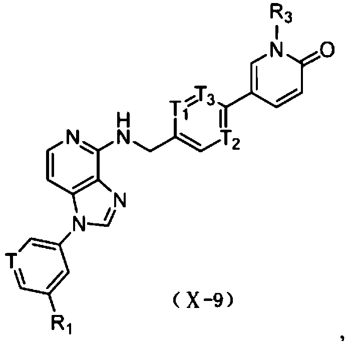 worin R₁, R₃, T, T₁, T₂ und T₃ wie in Anspruch 6 definiert sind.

8. Verbindung oder pharmazeutisch annehmbares Salz davon nach Anspruch 1, wobei die Verbindung aus den folgenden Formeln ausgewählt ist,

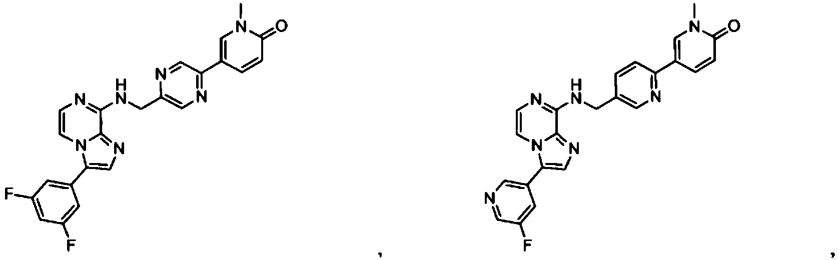

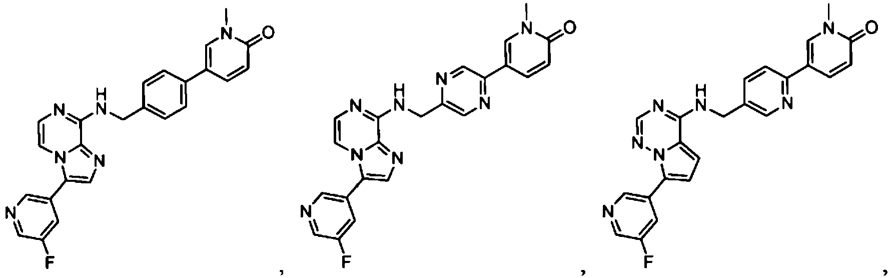

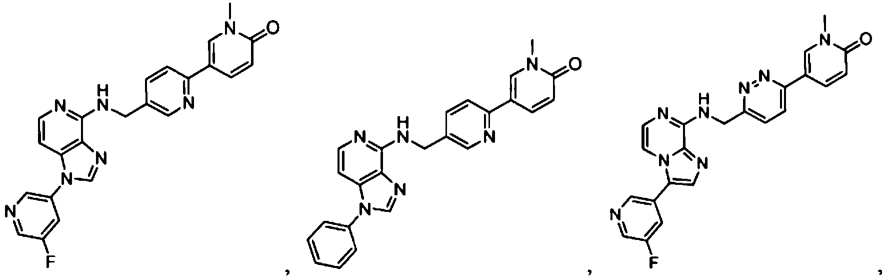

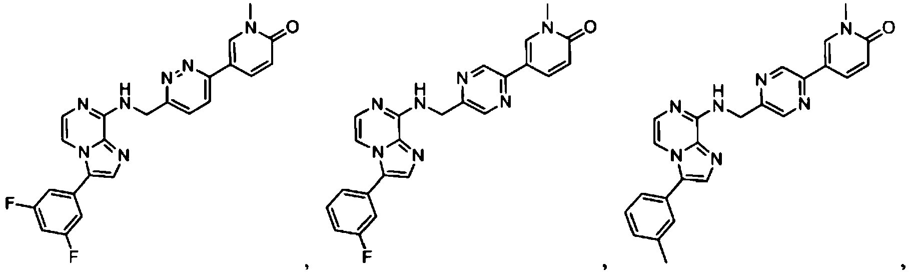

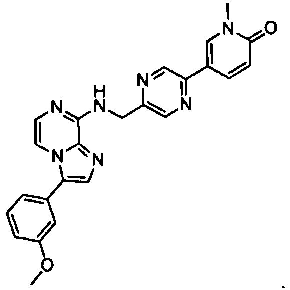

9. Kristallform A von Verbindung 2 nach Anspruch 8, wobei die Kristallform A ein Röntgenpulverbeugungsmuster aufweist, das charakteristische Beugungsreflexe bei folgenden 2θ-Winkeln umfasst: 7,45 ± 0,20°, 13,53 ± 0,20°, 13,94 ± 0,20° und 15,93 ± 0,20°;

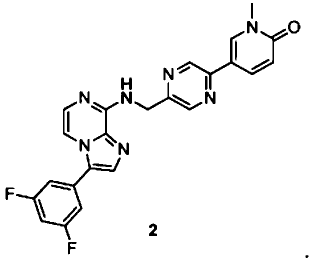

10. Kristallform A nach Anspruch 9, wobei das Röntgenpulverbeugungsmuster der Kristallform A charakteristische Beugungsreflexe bei folgenden 2θ-Winkeln umfasst: 7,45 ± 0,20°, 10,56 ± 0,20°, 13,53 ± 0,20°, 13,94 ± 0,20°, 14,86 ± 0,20°, 15,93 ± 0,20° und 17,96 ± 0,20°;
das Röntgenpulverbeugungsmuster der Kristallform A vorzugsweise charakteristische Beugungsreflexe bei folgenden 2θ-Winkeln umfasst:
4,96 ± 0,20°, 7,45 ± 0,20°, 10,56 ± 0,20°, 13,53 ± 0,20°, 13,94 ± 0,20°, 14,86 ± 0,20°, 15,93 ± 0,20°, 17,96 ± 0,20°, 19,87 ± 0,20°, 20,90 ± 0,20°, 25,38 ± 0,20° und 28,02 ± 0,20°;
das Röntgenpulverbeugungsmuster der Kristallform A noch bevorzugter charakteristische Beugungsreflexe bei folgenden 2θ-Winkeln umfasst:
4,96 ± 0,20°, 7,45 ± 0,20°, 10,56 ± 0,20°, 13,53 ± 0,20°, 13,94 ± 0,20°, 14,86 ± 0,20°, 15,93 ± 0,20°, 17,96 ± 0,20°, 19,06 ± 0,20°, 19,87 ± 0,20°, 20,90 ± 0,20°, 24,11 ± 0,20°, 24,97 ± 0,20°, 25,38 ± 0,20° und 28,02 ± 0,20°;
das Röntgenpulverbeugungsmuster der Kristallform A insbesondere charakteristische Beugungsreflexe bei folgenden 2θ-Winkeln umfasst:
4,96°, 7,45°, 10,56°, 13,53°, 13,94°, 14,86°, 15,93°, 17,56°, 17,96°, 19,06°, 19,87°, 20,90°, 21,21°, 21,90°, 22,63°, 24,11°, 24,97°, 25,38°, 25,96°, 27,14°, 28,02°, 28,73°, 29,94°, 30,89°, 32,50°, 34,01°, 35,05°, 35,95°, 37,54° und 39,08°.

11. Kristallform A nach Anspruch 9 oder 10, die XRPD-Musteranalysedaten wie in der folgenden Tabelle dargelegt aufweist:
| Nr. | 2θ-Winkel [°] | Reflexhöhe [cts] | Linke volle Breite bei halbem Maximum [°2θ] | Interplanare Abstände [Å] | Relative Intensität [%] |
|---|---|---|---|---|---|
| 1 | 4,96 | 798,52 | 0,1279 | 17,80 | 5,32 |
| 2 | 7,45 | 1349,94 | 0,1535 | 11,87 | 9,00 |
| 3 | 10,56 | 873,60 | 0,1791 | 8,37 | 5,83 |
| 4 | 13,53 | 4986,93 | 0,1791 | 6,55 | 33,25 |
| 5 | 13,94 | 14996,96 | 0,1791 | 6,35 | 100,00 |
| 6 | 14,86 | 1270,50 | 0,1791 | 5,96 | 8,47 |
| 7 | 15,93 | 3547,78 | 0,1791 | 5,56 | 23,66 |
| 8 | 17,56 | 432,50 | 0,1791 | 5,05 | 2,88 |
| 9 | 17,96 | 1770,00 | 0,1791 | 4,94 | 11,80 |
| 10 | 19,06 | 435,51 | 0,1535 | 4,66 | 2,90 |
| 11 | 19,87 | 899,45 | 0,2047 | 4,47 | 6,00 |
| 12 | 20,90 | 460,45 | 0,1535 | 4,25 | 3,07 |
| 13 | 21,21 | 423,35 | 0,1535 | 4,19 | 2,82 |
| 14 | 21,90 | 104,94 | 0,2558 | 4,06 | 0,70 |
| 15 | 22,63 | 291,20 | 0,1279 | 3,93 | 1,94 |
| 16 | 24,11 | 596,83 | 0,1791 | 3,69 | 3,98 |
| 17 | 24,97 | 871,02 | 0,2047 | 3,57 | 5,81 |
| 18 | 25,38 | 979,35 | 0,2047 | 3,51 | 6,53 |
| 19 | 25,96 | 879,75 | 0,1791 | 3,43 | 5,87 |
| 20 | 27,14 | 280,14 | 0,2047 | 3,29 | 1,87 |
| 21 | 28,02 | 877,39 | 0,2047 | 3,18 | 5,85 |
| 22 | 28,73 | 379,96 | 0,1535 | 3,11 | 2,53 |
| 23 | 29,94 | 275,89 | 0,4093 | 2,98 | 1,84 |
| 24 | 30,89 | 224,53 | 0,2047 | 2,89 | 1,50 |
| 25 | 32,50 | 309,77 | 0,2047 | 2,75 | 2,07 |
| 26 | 34,01 | 178,62 | 0,2558 | 2,64 | 1,19 |
| 27 | 35,05 | 248,70 | 0,1535 | 2,56 | 1,66 |
| 28 | 35,95 | 67,09 | 0,3070 | 2,50 | 0,45 |
| 29 | 37,54 | 224,08 | 0,2303 | 2,40 | 1,49 |
| 30 | 39,08 | 170,32 | 0,2047 | 2,30 | 1,14 |

12. Kristallform A nach einem der Ansprüche 9 bis 11, wobei die Kristallform A bei dynamischer Differenzkalorimetrie (DSC) eine Kurve aufweist, die den Beginn endothermer Peaks bei 259,7 °C ± 5 °C und 274,7 °C ± 5 °C umfasst.

13. Kristallform A nach einem der Ansprüche 9 bis 11, wobei die Kristallform A eine bei thermogravimetrischer Analyse (TGA) eine Kurve mit einem Gewichtsverlust von 0,80 % bei 240 °C ± 3 °C aufweist.

14. Verbindung oder pharmazeutisch annehmbares Salz davon nach einem der Ansprüche 1 bis 8 oder Kristallform A nach einem der Ansprüche 9 bis 13 zur Verwendung bei der Inhibierung des Porcupine-Proteins.

15. Verbindung oder pharmazeutisch annehmbares Salz davon nach einem der Ansprüche 1 bis 8 oder Kristallform A nach einem der Ansprüche 9 bis 13 zur Verwendung bei der Behandlung von Pankreaskrebs, Kolorektalrekbs und/oder Magenkrebs.

## Revendications

1. Composé de formule (X), ou sel pharmaceutiquement acceptable de celui-ci, 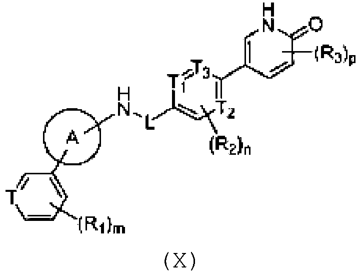 dans lequel
T est choisi parmi CR ou N ;
T₁ est choisi parmi CH ou N ;
T₂ est choisi parmi CH ou N ;
T₃ est choisi parmi CH ou N ;
L est choisi parmi une liaison simple et -C(R₄R₅)- ;
le cycle A est un hétéroaryle de 9 à 10 termes, l'hétéroaryle de 9 à 10 termes étant facultativement substitué par 1, 2 ou 3 Rₐ ;
chaque R₁ est choisi indépendamment parmi H, F, Cl, Br, I, -CN, -OH, -NH₂, alkyle en C₁₋₃, alcoxy en C₁₋₃ et alkylamino en C₁₋₃, l'alkyle en C₁₋₃, l'alcoxy en C₁₋₃ et l'alkylamino en C₁₋₃ étant chacun indépendamment et facultativement substitués par 1, 2 ou 3 R_{b} ;
chaque R₂ est choisi indépendamment parmi H, F, Cl, Br, I, -CN, -OH, -NH₂, alkyle en C₁₋₃, alcoxy en C₁₋₃ et alkylamino en C₁₋₃, l'alkyle en C₁₋₃, l'alcoxy en C₁₋₃ et l'alkylamino en C₁₋₃ étant chacun indépendamment et facultativement substitués par 1, 2 ou 3 R_{c} ;
chaque R₃ est choisi indépendamment parmi H, F, Cl, Br, I, -CN, -OH, -NH₂, alkyle en C₁₋₃, alcoxy en C₁₋₃ et alkylamino en C₁₋₃, l'alkyle en C₁₋₃, l'alcoxy en C₁₋₃ et l'alkylamino en C₁₋₃ étant chacun indépendamment et facultativement substitués par 1, 2 ou 3 R_{d} ;
R₄ et R₅ sont choisis chacun indépendamment parmi H et alkyle en C₁₋₃, l'alkyle en C₁₋₃ étant facultativement substitué par 1, 2 ou 3 Rₑ ;
R est choisi parmi H, F, Cl, Br, I, -CN, -OH, -NH₂, alkyle en C₁₋₃, alcoxy en C₁₋₃ et alkylamino en C₁₋₃, l'alkyle en C₁₋₃, l'alcoxy en C₁₋₃ et l'alkylamino en C₁₋₃ étant chacun indépendamment et facultativement substitués par 1, 2 ou 3 R_{f} ;
chaque Rₐ est choisi indépendamment parmi F, Cl, Br, I, -OH, -NH₂ et -CN ;
chaque R_{b} est choisi indépendamment parmi F, Cl, Br, I, -OH, -NH₂ et -CN ;
chaque R_{c} est choisi indépendamment parmi F, Cl, Br, I, -OH, -NH₂ et -CN ;
chaque R_{d} est choisi indépendamment parmi F, Cl, Br, I, -OH, -NH₂ et -CN ;
chaque Rₑ est choisi indépendamment parmi F, Cl, Br, I, -OH, -NH₂ et -CN ;
chaque R_{f} est choisi indépendamment parmi F, Cl, Br, I, -OH, -NH₂ et -CN ;
m est choisi parmi 0, 1, 2 et 3 ;
n est choisi parmi 0, 1, 2 et 3 ;
p est choisi parmi 0, 1, 2 et 3 ;
"hétéro" dans l'hétéroaryle de 9 à 10 termes représente 1, 2, 3 ou 4 hétéroatomes ou groupes d'hétéroatomes choisis indépendamment parmi O, S et N.

2. Composé ou sel pharmaceutiquement acceptable de celui-ci selon la revendication 1, dans lequel le composé a une structure de formule (X-1) : 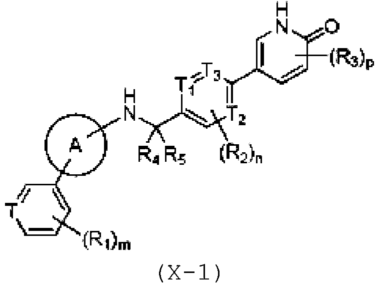 dans lequel le cycle A, R₁, R₂, R₃, R₄, R₅, T, T₁, T₂, T₃, m, n et p sont tels que définis dans la revendication 1.

3. Composé ou sel pharmaceutiquement acceptable de celui-ci selon la revendication 1 ou la revendication 2, dans lequel
chaque R₁ est choisi indépendamment parmi H, F, Cl, Br, I, -CN, -OH, -NH₂, -CH₃ et -OCH₃, le -CH₃ et le -OCH₃ étant chacun indépendamment et facultativement substitués par 1, 2 ou 3 R_{b} ;
ou chaque R₂ est choisi indépendamment parmi H ;
ou chaque R₃ est choisi indépendamment parmi H, F, Cl, Br, I, -CN, -OH, -NH₂ et -CH₃, le -CH₃ étant facultativement substitué par 1, 2 ou 3 R_{d} ;
ou R₄ et R₅ sont choisis chacun indépendamment parmi H et -CH₃, le -CH₃ étant facultativement substitué par 1, 2 ou 3 Rₑ ;
ou R est choisi parmi H, F, Cl, Br, I, -CN, -OH, -NH₂ et -CH₃, le -CH₃ étant facultativement substitué par 1, 2 ou 3 R_{f} ;
ou le cycle A est choisi parmi 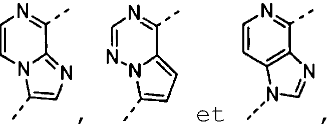 où 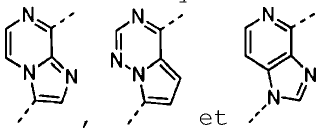 sont chacun indépendamment et facultativement substitués par 1, 2 ou 3 Rₐ.

4. Composé ou sel pharmaceutiquement acceptable de celui-ci selon la revendication 3, dans lequel chaque R₁ est choisi indépendamment parmi H, F, -CH₃ et -OCH₃ ;
ou chaque R₃ est choisi indépendamment parmi -CH₃ ;
ou R₄ et R₅ sont choisis chacun indépendamment parmi H ;
ou R est choisi parmi H et F ;
ou le cycle A est choisi parmi

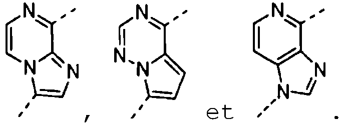

5. Composé ou sel pharmaceutiquement acceptable de celui-ci selon la revendication 1 ou la revendication 2, dans lequel le composé a une structure de formule (X-2) ou (X-3) : 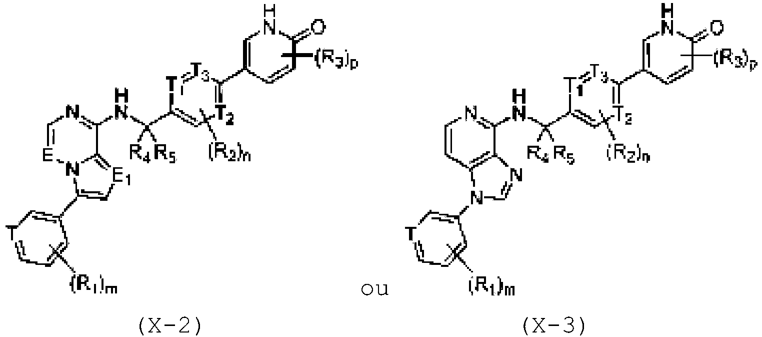 dans laquelle
E est choisi parmi CH et N ;
E₁ est choisi parmi CH et N ;
R₁, R₂, R₃, R₄, R₅, T, T₁, T₂, T₃, m, n et p sont tels que définis dans la revendication 1 ou la revendication 2.

6. Composé ou sel pharmaceutiquement acceptable de celui-ci selon la revendication 5, dans lequel le composé a une structure de formule (X-4), (X-5) ou (X-6) : 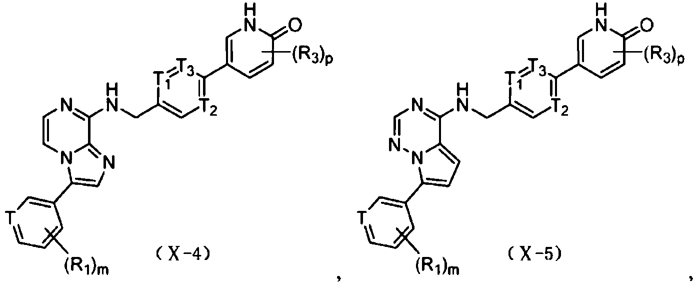 ou 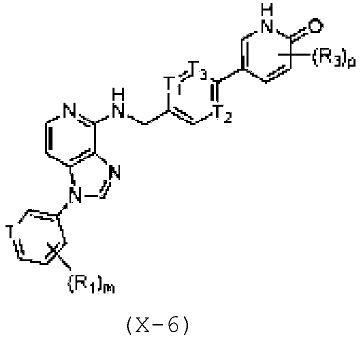 où R₁, R₃, T, T₁, T₂, T₃, m et p sont tels que définis dans la revendication 5.

7. Composé ou sel pharmaceutiquement acceptable de celui-ci selon la revendication 6, dans lequel le composé a une structure de formules (X-7), (X-8) et (X-9) : 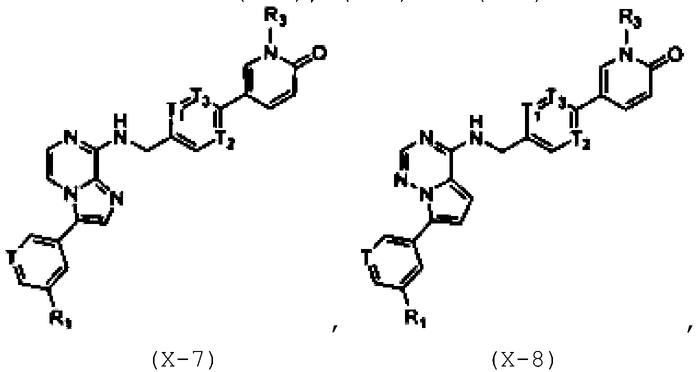 ou 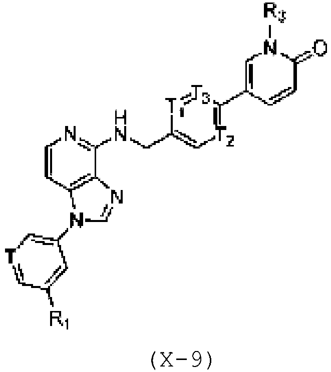 où R₁, R₃, T, T₁, T₂ et T₃ sont tels que définis dans la revendication 6.

8. Composé ou sel pharmaceutiquement acceptable de celui-ci selon la revendication 1, dans lequel le composé est choisi parmi les formules suivantes :

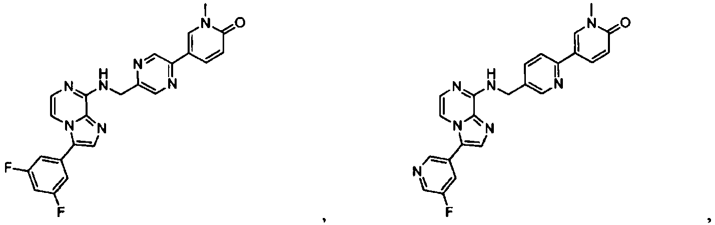

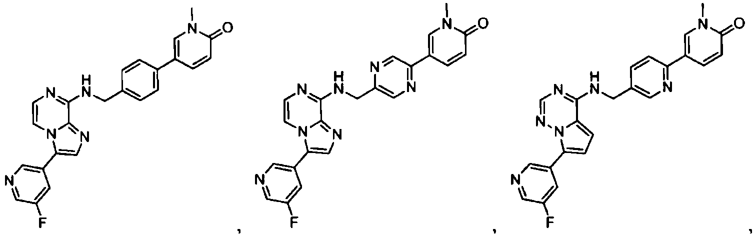

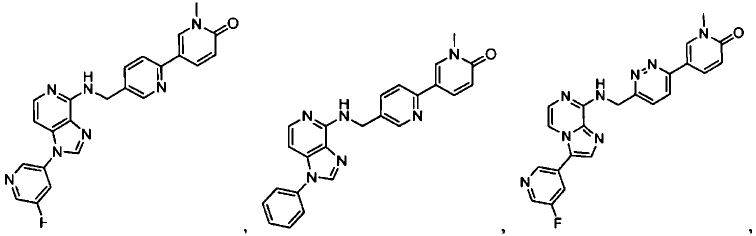

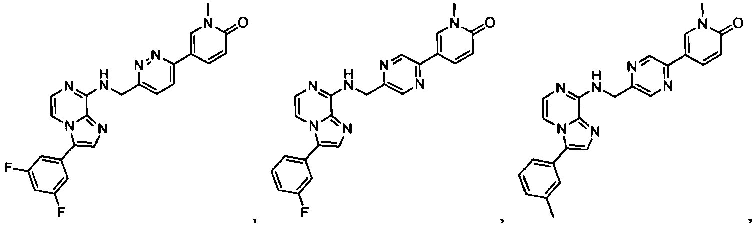

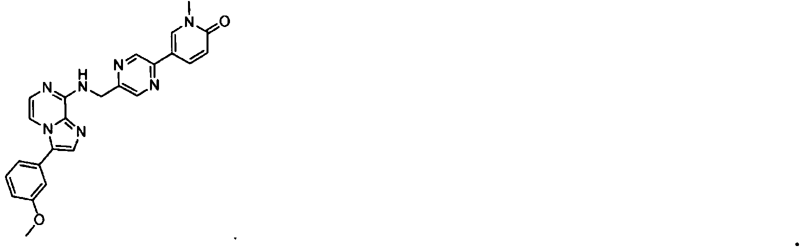

9. Forme cristalline A du composé 2 selon la revendication 8, dans laquelle la forme cristalline A présente un diagramme de diffraction des rayons X sur poudre comprenant des pics de diffraction caractéristiques aux angles 2θ suivants : 7,45 ± 0,20°, 13,53 ± 0,20°, 13,94 ± 0,20° et 15,93 ± 0,20° ;

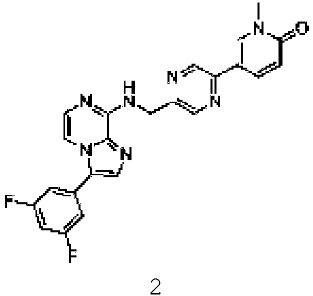

10. Forme cristalline A selon la revendication 9, dans laquelle le diagramme de diffraction des rayons X sur poudre de la forme cristalline A comprend des pics de diffraction caractéristiques aux angles 2θ suivants : 7,45 ± 0,20°, 10,56 ± 0,20°, 13,53 ± 0,20°, 13,94 ± 0,20°, 14,86 ± 0,20°, 15,93 ± 0,20° et 17,96 ± 0,20° ;
de préférence, le diagramme de diffraction des rayons X sur poudre de la forme cristalline A comprend des pics de diffraction caractéristiques aux angles 2θ suivants : 4,96 ± 0,20°, 7,45 ± 0,20°, 10,56 ± 0,20°, 13,53 ± 0,20°, 13,94 ± 0,20°, 14,86 ± 0,20°, 15,93 ± 0,20°, 17,96 ± 0,20°, 19,87 ± 0,20°, 20,90 ± 0,20°, 25,38 ± 0,20° et 28,02 ± 0,20° ;
plus préférentiellement, le diagramme de diffraction des rayons X sur poudre de la forme cristalline A comprend des pics de diffraction caractéristiques aux angles 2θ suivants : 4,96 ± 0,20°, 7,45 ± 0,20°, 10,56 ± 0,20°, 13,53 ± 0,20°, 13,94 ± 0,20°, 14,86 ± 0,20°, 15,93 ± 0,20°, 17,96 ± 0,20°, 19,06 ± 0,20°, 19,87 ± 0,20°, 20,90 ± 0,20°, 24,11 ± 0,20°, 24,97 ± 0,20°, 25,38 ± 0,20° et 28,02 ± 0,20° ;
encore plus préférentiellement, le diagramme de diffraction des rayons X sur poudre de la forme cristalline A comprend des pics de diffraction caractéristiques aux angles 2θ suivants : 4,96°, 7,45°, 10,56°, 13,53°, 13,94°, 14,86°, 15,93°, 17,56°, 17,96°, 19,06°, 19,87°, 20,90°, 21,21°, 21,90°, 22,63°, 24,11°, 24,97°, 25,38°, 25,96°, 27,14°, 28,02°, 28,73°, 29,94°, 30,89°, 32,50°, 34,01°, 35,05°, 35,95°, 37,54° et 39,08°.

11. Forme cristalline A selon la revendication 9 ou la revendication 10, présentant des données d'analyse du diagramme XRPD comme indiqué dans le tableau suivant :
| N° | Angle 2θ [°] | Hauteur du pic [cts] | À gauche, largeur maximale à mi-hauteur [°2θ] | Distance inter-planaire [Å] | Intensité relative [%] |
|---|---|---|---|---|---|
| 1 | 4,96 | 798,52 | 0,1279 | 17,80 | 5,32 |
| 2 | 7,45 | 1349,94 | 0,1535 | 11,87 | 9,00 |
| 3 | 10,56 | 873,60 | 0,1791 | 8,37 | 5,83 |
| 4 | 13,53 | 4986,93 | 0,1791 | 6,55 | 33,25 |
| 5 | 13,94 | 14996,96 | 0,1791 | 6,35 | 100,00 |
| 6 | 14,86 | 1270,50 | 0,1791 | 5,96 | 8,47 |
| 7 | 15,93 | 3547,78 | 0,1791 | 5,56 | 23,66 |
| 8 | 17,56 | 432,50 | 0,1791 | 5,05 | 2,88 |
| 9 | 17,96 | 1770,00 | 0,1791 | 4,94 | 11,80 |
| 10 | 19,06 | 435,51 | 0,1535 | 4,66 | 2,90 |
| 11 | 19,87 | 899,45 | 0,2047 | 4,47 | 6,00 |
| 12 | 20,90 | 460,45 | 0,1535 | 4,25 | 3,07 |
| 13 | 21,21 | 423,35 | 0,1535 | 4,19 | 2,82 |
| 14 | 21,90 | 104,94 | 0,2558 | 4,06 | 0,70 |
| 15 | 22,63 | 291,20 | 0,1279 | 3,93 | 1,94 |
| 16 | 24,11 | 596,83 | 0,1791 | 3,69 | 3,98 |
| 17 | 24,97 | 871,02 | 0,2047 | 3,57 | 5,81 |
| 18 | 25,38 | 979,35 | 0,2047 | 3,51 | 6,53 |
| 19 | 25,96 | 879,75 | 0,1791 | 3,43 | 5,87 |
| 20 | 27,14 | 280,14 | 0,2047 | 3,29 | 1,87 |
| 21 | 28,02 | 877,39 | 0,2047 | 3,18 | 5,85 |
| 22 | 28,73 | 379,96 | 0,1535 | 3,11 | 2,53 |
| 23 | 29,94 | 275,89 | 0,4093 | 2,98 | 1,84 |
| 24 | 30,89 | 224,53 | 0,2047 | 2,89 | 1,50 |
| 25 | 32,50 | 309,77 | 0,2047 | 2,75 | 2,07 |
| 26 | 34,01 | 178,62 | 0,2558 | 2,64 | 1,19 |
| 27 | 35,05 | 248,70 | 0,1535 | 2,56 | 1,66 |
| 28 | 35,95 | 67,09 | 0,3070 | 2,50 | 0,45 |
| 29 | 37,54 | 224,08 | 0,2303 | 2,40 | 1,49 |
| 30 | 39,08 | 170,32 | 0,2047 | 2,30 | 1,14 |

12. Forme cristalline A selon l'une quelconque des revendications 9 à 11, dans laquelle la forme cristalline A présente une courbe de calorimétrie différentielle à balayage (DSC) comprenant des débuts de pics endothermiques à 259,7 °C ± 5 °C et 274,7 °C ± 5 °C.

13. Forme cristalline A selon l'une quelconque des revendications 9 à 11, dans laquelle la forme cristalline A présente une courbe d'analyse thermogravimétrique (TGA) avec une perte de poids de 0,80 % à 240 °C ± 3 °C.

14. Composé ou sel pharmaceutiquement acceptable de celui-ci selon l'une quelconque des revendications 1 à 8, ou forme cristalline A selon l'une quelconque des revendications 9 à 13, destiné(e) à être utilisé(e) dans l'inhibition de la porcupine.

15. Composé ou sel pharmaceutiquement acceptable de celui-ci selon l'une quelconque des revendications 1 à 8, ou forme cristalline A selon l'une quelconque des revendications 9 à 13, destiné(e) à être utilisé(e) dans le traitement du cancer du pancréas, du cancer colorectal et/ou du cancer de l'estomac.
